# EUROPEAN PATENT SPECIFICATION

(11) **EP 2 744 891 B1**
(45) Date of publication and mention of the grant of the patent: **12.12.2018**
(21) Application number: 12825305.1
(22) Date of filing: 20.08.2012
(51) Int. Cl.: C12N 5/071, C12N 5/02

(54) **NEUROGENIC AND GLIOGENIC FACTORS AND ASSAYS THEREFOR**
NEUROGENE UND GLIOGENE FAKTOREN SOWIE ASSAYS DAFÜR
FACTEURS NEUROGÈNES ET GLIOGÈNES ET LEURS DOSAGES

(30) Priority: 19.08.2011 US 201161575378 P; 28.12.2011 US 201161580991 P
(43) Date of publication of application: 25.06.2014
(73) Proprietor: SanBio, Inc., Mountain View, CA 94041-1522 (US)
(72) Inventor: AIZMAN, Irina, Mountain View, CA 94041 (US); CASE, Casey, C., San Mateo, CA 94402 (US)
(74) Representative: Patentanwälte Bals & Vogel
(86) International application number: PCT/US2012/051596
(87) International publication number: WO 2013/028625

(56) References cited:
- WO-A1-2009/023251
- WO-A1-2009/073178
- WO-A2-2007/008758
- US-A1- 2004 185 429
- AIZMAN ET AL.: 'Extracellular matrix produced by bone marrow stromal cells and by their derivative, SB623 cells, supports neural cell growth' J NEUROSCI RES vol. 87, no. 14, 01 November 2009, pages 3198 - 3206, XP002634939
- ADAM HARVEY ET AL: "Proteomic Analysis of the Extracellular Matrix Produced by Mesenchymal Stromal Cells: Implications for Cell Therapy Mechanism", PLOS ONE, vol. 8, no. 11, 14 November 2013 (2013-11-14), page e79283, XP055233633, DOI: 10.1371/journal.pone.0079283

## Description

### FIELD

This application is in the field of substances that promote neurogenesis and gliogenesis, and assays for such substances.

### BACKGROUND

Mesenchymal stromal cells contain a population of multipotent cells, known as mesenchymal stem cells (reviewed in [1]). A major source of mesenchymal stem cells (MSC) in adult mammals is the bone marrow; multipotent cells obtained from bone marrow are known variously as mesenchymal stem cells (MSC), marrow adherent stromal cells (MASC), marrow adherent stem cells, and bone marrow stromal cells (BMSC). Mesenchymal stem cells have been studied as a potential cellular therapy for the repair of neural tissue (reviewed in [2]). Transplantation of MSC or MSC derivatives into the nervous system has been shown to be beneficial in many models of neurodegenerative diseases including stroke, Parkinson's disease, spinal cord injury, multiple sclerosis, and neonatal hypoxic-ischemic brain injury [3-9].

Current evidence suggests that the transplantation of MSC or their derivatives activates endogenous regeneration mechanisms both in injured neural tissue [9-13] and in normal brain tissue [14]. These regenerative processes include enhanced proliferation of endogenous neural stem cells, increased survival of newborn neurons [10-11], gliogenesis [7], and modulation of inflammatory cytokine production [15]. It is thought that the neuroprotection and enhancement of neural proliferation are mediated, at least in part, by diffusible neurotrophic factors and cytokines secreted by the transplanted cells. Indeed, MSC have been shown to secrete a number of growth factors in culture [16, 17]; and the identity of the growth factors secreted can be modulated by transplantation in a neurodegenerative environment [18, 19].

It is thus important to identify the factors produced by MSC, and their derivatives, that are responsible for the neuropoietic and gliogenic activities of mesenchymal cells. The study of interactions between MSC and neural cells *in vitro* poses the challenge of creating culture conditions that are suitable for several different cell types (*e.g.,* neurons, glial cells, neural stem cells, mesenchymal cells), each having different requirements for substrate and growth media. Indeed, in most systems, co-culture conditions (for example, the presence or absence of serum, or the use of MSC monolayers as substrate for small numbers of neural cells) selectively favor certain cells at the expense of others, which leads to inconsistent results [23-26] and prevents the adequate quantification of MSC effects. For example, certain culture systems are favorable to the growth of neurons, but not of glial cells; and no system has been found that supports the growth of neural precursor cells and the three major types of neural cell (neuron, oligodendrocyte and astrocyte) simultaneously.

The effects of MSC and other substances on proliferation and differentiation of neural stem cells into various neural lineages (*i.e.,* neuropoiesis) are commonly studied *in vitro* using mitogen-driven neurospheres as a source of neural stem/early precursor cells; subsequently, their differentiation is induced by plating neurospheres on an adhesive substrate and withdrawing the mitogenic growth factors [23-26]. However, cells in neurospheres may not reflect a natural pool of neural precursors because their growth conditions select for responders to non-physiologically high concentrations of growth factors and unattached growth [27, 28]. It is thus possible that by the beginning of co-culturing, cells derived from neurospheres may have been reprogrammed by the culture conditions. Furthermore, in neurosphere co-culture experiments the state of growth of neural stem cell progenitors is difficult to observe because it occurs in a "blind spot" within the neurospheres themselves. Finally, induction of neural differentiation through the change of cell attachment status may obscure the effects of test substances, in the neurosphere system.

For the reasons stated above, systems capable of quantifying the effects of neurogenic and gliogenic factors on neural precursor cells, neurons, astrocytes and oligodendrocytes under the same conditions have not been available.

SB623 cells are derived from MSC by transfecting MSC with a vector encoding a Notch1 intracellular domain. See, *e.g.,* U.S. Patent No. 7,682,825. Previous work has shown that ECM produced by human MSC, and SB623 cells derived therefrom, effectively supports the growth and differentiation of rat embryonic cortical cells without added factors or serum (29, see also US 2010/0310529, which describes certain properties of the ECM produced by MSC and SB623 cells).

As set forth above, there remains a need for a simple and accurate *in vitro* system that models the interactions of substances possessing neurogenic and/or gliogenic activity (*e.g.,* MSC and their derivatives, *e.g.,* SB623 cells) with complex populations of neural cells, and quantifies the potency of such substances.

### SUMMARY

Provided herein are *in vitro* systems for co-culture of MSC, and/or their derivatives (e.g., SB623 cells), with neural cell populations under conditions that optimize the ability to quantitate the effects of factors that influence the growth and differentiation of the different neural cells in the culture.

These culture systems can be used to provide quantitative functional assays for measuring the effects of substances (*e.g.,* MSC and their derivatives, *e.g.,* SB623 cells, conditioned medium, polypeptides, organic compounds) on various types of neural cells (*e.g.* neurons, astrocytes, oligodendrocytes). In particular, neurotrophic, neurogenic, gliotrophic and gliogenic factors, and sources of such factors, can be identified and quantitated.

Using the assays described herein, a number of substances having neurogenic and gliogenic activity have been identified.

Accordingly, the present disclosure provides, *inter alia,* the following embodiments.
**1.** A method for testing for a substance that promotes neurogenesis, the method comprising:
   (a) culturing mesenchymal stem cells (MSC) on a solid substrate;
   (b) removing the MSC from the substrate, such that an extracellular matrix produced by the MSC remains on the substrate;
   (c) culturing embryonic cortical cells on the substrate of step (b);
   (d) adding a substance to the culture of step (c); and
   (e) measuring growth of neurons;
   wherein growth of neurons indicates that the substance promotes neurogenesis.
**2.** The method of embodiment 1, wherein the MSC are obtained from a human.
**3.** The method of embodiment 1, wherein the solid substrate is selected from the group consisting of plastic, nitrocellulose and glass.
**4.** The method of embodiment 1, wherein the embryonic cortical cells are obtained from a mouse or a rat.
**5.** The method of embodiment 1, wherein the substance is a chemical compound or a polypeptide.
**6.** The method of embodiment 1, wherein the substance is a cell or a cell culture. In certain embodiments, the cell is a mesenchymal stem cell. In additional embodiments, the cell is a descendant of a mesenchymal stem cell that has been transfected with a nucleic acid encoding a Notch intracellular domain (a SB623 cell).
**7.** The method of embodiment 6, wherein the neurogenesis is promoted by a protein expressed on the surface of the cell.
**8.** The method of embodiment 1, wherein the substance is a conditioned medium from a cell culture.
**9.** The method of embodiment 1, wherein growth of neurons is measured by neurite outgrowth or by expression of a marker selected from the group consisting of microtubule-associated protein 2 (MAP2), doublecortin (DCX), beta-tubulin type III (TuJ1), synaptophysin and neuron-specific enolase.
**10.** The method of embodiment 1, wherein growth of neurons is compared to growth of neurons in the absence of the substance.
**11.** A method for testing for a substance that promotes gliogenesis, the method comprising:
   (a) culturing mesenchymal stem cells (MSC) on a solid substrate;
   (b) removing the MSC from the substrate, such that an extracellular matrix produced by the MSC remains on the substrate;
   (c) culturing embryonic cortical cells on the substrate of step (b);
   (d) adding a substance to the culture of step (c); and
   (e) measuring growth of glial cells;
   wherein growth of glial cells indicates that the substance promotes gliogenesis.
**12.** The method of embodiment 11, wherein the MSC are obtained from a human.
**13.** The method of embodiment 11, wherein the solid substrate is selected from the group consisting of plastic, nitrocellulose and glass.
**14.** The method of embodiment 11, wherein the embryonic cortical cells are obtained from a mouse or a rat.
**15.** The method of embodiment 11, wherein the substance is a chemical compound or a polypeptide.
**16.** The method of embodiment 11, wherein the substance is a cell or a cell culture. In certain embodiments, the cell is a mesenchymal stem cell. In additional embodiments, the cell is a descendant of a mesenchymal stem cell that has been transfected with a nucleic acid encoding a Notch intracellular domain (a SB623 cell).
**17.** The method of embodiment 16, wherein the gliogenesis is promoted by a protein expressed on the surface of the cell.
**18.** The method of embodiment 11, wherein the substance is a conditioned medium from a cell culture.
**19.** The method of embodiment 11, wherein the growth of glial cells is compared to growth of glial cells in the absence of the substance.
**20.** The method of embodiment 11, wherein the glial cells are astrocytes.
**21.** The method of embodiment 20, wherein growth of the astrocytes is measured by expression of glial fibrillary acidic protein (GFAP), Glast, or glutamine synthetase.
**22.** The method of embodiment 11,wherein the glial cells are oligodendrocytes.
**23.** The method of embodiment 22, wherein growth of the oligodendrocytes is measured by expression a marker selected from the group consisting of 2', 3'-cyclic nucleotide 3' phosphodiesterase (CNPase), the O1 antigen, the 04 antigen, myelin basic protein, oligodendrocyte transcription factor 1, oligodendrocyte transcription factor 2, oligodendrocyte transcription factor 3, NG2, and myelin-associated glycoprotein.
**24.** A method for testing for a substance that promotes neurogenesis, the method comprising:
   (a) culturing cells on a solid substrate, wherein the cells are descendants of mesenchymal stem cells that have been transfected with a nucleic acid encoding a Notch intracellular domain;
   (b) removing the cells from the substrate,
   (c) culturing embryonic cortical cells on the substrate of step (b);
   (d) adding a substance to the culture of step (c); and
   (e) measuring growth of neurons;
   wherein growth of neurons indicates that the substance promotes neurogenesis.
**25.** The method of embodiment 24, wherein the MSC are obtained from a human.
**26.** The method of embodiment 24, wherein the solid substrate is selected from the group consisting of plastic, nitrocellulose and glass.
**27.** The method of embodiment 24, wherein the embryonic cortical cells are obtained from a mouse or a rat.
**28.** The method of embodiment 24, wherein the substance is a chemical compound or a polypeptide.
**29.** The method of embodiment 24, wherein the substance is a cell or a cell culture. In certain embodiments, the cell is a mesenchymal stem cell. In additional embodiments, the cell is a descendant of a mesenchymal stem cell that has been transfected with a nucleic acid encoding a Notch intracellular domain (a SB623 cell).
**30.** The method of embodiment 29, wherein the neurogenesis is promoted by a protein expressed on the surface of the cell.
**31.** The method of embodiment 24, wherein the substance is a conditioned medium from a cell culture.
**32.** The method of embodiment 24, wherein growth of neurons is measured by neurite outgrowth or by expression of a marker selected from the group consisting of microtubule-associated protein 2 (MAP2), doublecortin (DCX), beta-tubulin type III (TuJ1), synaptophysin and neuron-specific enolase.
**33.** The method of embodiment 24, wherein growth of neurons is compared to growth of neurons in the absence of the substance.
**34.** A method for testing for a substance that promotes gliogenesis, the method comprising:
   (a) culturing cells on a solid substrate, wherein the cells are descendants of mesenchymal stem cells that have been transfected with a nucleic acid encoding a Notch intracellular domain;
   (b) removing the cells from the substrate, such that an extracellular matrix produced by the cells remains on the substrate;
   (c) culturing embryonic cortical cells on the substrate of step (b);
   (d) adding a substance to the culture of step (c); and
   (e) measuring growth of glial cells;
   wherein growth of glial cells indicates that the substance promotes gliogenesis.
**35.** The method of embodiment 34, wherein the MSC are obtained from a human.
**36.** The method of embodiment 34, wherein the solid substrate is selected from the group consisting of plastic, nitrocellulose and glass.
**37.** The method of embodiment 34, wherein the embryonic cortical cells are obtained from a mouse or a rat.
**38.** The method of embodiment 34, wherein the substance is a chemical compound or a polypeptide.
**39.** The method of embodiment 34, wherein the substance is a cell or a cell culture. In certain embodiments, the cell is a mesenchymal stem cell. In additional embodiments, the cell is a descendant of a mesenchymal stem cell that has been transfected with a nucleic acid encoding a Notch intracellular domain (a SB623 cell).
**40.** The method of embodiment 34, wherein the gliogenesis is promoted by a protein expressed on the surface of the cell.
**41.** The method of embodiment 34, wherein the substance is a conditioned medium from a cell culture.
**42.** The method of embodiment 34, wherein the growth of glial cells is compared to growth of glial cells in the absence of the substance.
**43.** The method of embodiment 34, wherein the glial cells are astrocytes.
**44.** The method of embodiment 43, wherein growth of the astrocytes is measured by expression of glial fibrillary acidic protein (GFAP), Glast, or glutamine synthetase.
**45.** The method of embodiment 34,wherein the glial cells are oligodendrocytes.
**46.** The method of embodiment 45, wherein growth of the oligodendrocytes is measured by expression a marker selected from the group consisting of 2', 3'-cyclic nucleotide 3' phosphodiesterase (CNPase), the O1 antigen, the 04 antigen, myelin basic protein, oligodendrocyte transcription factor 1, oligodendrocyte transcription factor 2, oligodendrocyte transcription factor 3, NG2, and myelin-associated glycoprotein.
**47.** A method for testing for a substance that promotes the growth of neural precursor cells (NPC), the method comprising:
   (a) culturing mesenchymal stem cells (MSC) on a solid substrate;
   (b) removing the MSC from the substrate, such that an extracellular matrix produced by the MSC remains on the substrate;
   (c) culturing embryonic cortical cells on the substrate of step (b);
   (d) adding a substance to the culture of step (c); and
   (e) measuring growth of neural precursor cells;
   wherein growth of NPC indicates that the substance promotes the growth of NPC.
**48.** The method of embodiment 47, wherein the MSC are obtained from a human.
**49.** The method of embodiment 47, wherein the solid substrate is selected from the group consisting of plastic, nitrocellulose and glass.
**50.** The method of embodiment 47, wherein the embryonic cortical cells are obtained from a mouse or a rat.
**51.** The method of embodiment 47, wherein the substance is a chemical compound or a polypeptide.
**52.** The method of embodiment 47, wherein the substance is a cell or a cell culture. In certain embodiments, the cell is a mesenchymal stem cell. In additional embodiments, the cell is a descendant of a mesenchymal stem cell that has been transfected with a nucleic acid encoding a Notch intracellular domain (a SB623 cell).
**53.** The method of embodiment 52, wherein the growth of neural precursor cells is promoted by a protein expressed on the surface of the cell.
**54.** The method of embodiment 47, wherein the substance is a conditioned medium from a cell culture.
**55.** The method of embodiment 47, wherein growth of NPC is measured by expression of nestin or SOX2.
**56.** The method of embodiment 47, wherein growth of NPC is compared to growth of NPC in the absence of the substance.
**57.** A method for testing for a substance that promotes the growth of neural precursor cells (NPC), the method comprising:
   (a) culturing cells on a solid substrate, wherein the cells are descendants of mesenchymal stem cells (MSC) that have been transfected with a nucleic acid encoding a Notch intracellular domain;
   (b) removing the cells from the substrate, such that an extracellular matrix produced by the cells remains on the substrate;
   (c) culturing embryonic cortical cells on the substrate of step (b);
   (d) adding a substance to the culture of step (c); and
   (e) measuring growth of NPC;
   wherein growth of NPC indicates that the substance promotes growth of NPC.
**58.** The method of embodiment 57, wherein the MSC are obtained from a human.
**59.** The method of embodiment 57, wherein the solid substrate is selected from the group consisting of plastic, nitrocellulose and glass.
**60.** The method of embodiment 57, wherein the embryonic cortical cells are obtained from a mouse or a rat.
**61.** The method of embodiment 57, wherein the substance is a chemical compound or a polypeptide.
**62.** The method of embodiment 57, wherein the substance is a cell or a cell culture. In certain embodiments, the cell is a mesenchymal stem cell. In additional embodiments, the cell is a descendant of a mesenchymal stem cell that has been transfected with a nucleic acid encoding a Notch intracellular domain (a SB623 cell).
**63.** The method of embodiment 62, wherein the growth of neural precursor cells is promoted by a protein expressed on the surface of the cell.
**64.** The method of embodiment 57, wherein the substance is a conditioned medium from a cell culture.
**65.** The method of embodiment 57, wherein growth of NPC is measured by expression of nestin, Glast or SOX2.
**66.** The method of embodiment 57, wherein growth of NPC is compared to growth of NPC in the absence of the substance.
**67.** A method for testing for a substance that promotes the differentiation of neural precursor cells (NPC), the method comprising:
   (a) culturing mesenchymal stem cells (MSC) on a solid substrate;
   (b) removing the MSC from the substrate, such that an extracellular matrix produced by the MSC remains on the substrate;
   (c) culturing embryonic cortical cells on the substrate of step (b);
   (d) adding a substance to the culture of step (c); and
   (e) measuring differentiation of NPC;
   wherein differentiation of NPC indicates that the substance promotes the differentiation of NPC.
**68.** A method for testing for a substance that promotes the differentiation of neural precursor cells (NPC), the method comprising:
   (a) culturing cells on a solid substrate, wherein the cells are descendants of mesenchymal stem cells (MSC) that have been transfected with a nucleic acid encoding a Notch intracellular domain;
   (b) removing the cells from the substrate, such that an extracellular matrix produced by the cells remains on the substrate;
   (c) culturing embryonic cortical cells on the substrate of step (b);
   (d) adding a substance to the culture of step (c); and
   (e) measuring differentiation of NPC;
   wherein differentiation of NPC indicates that the substance promotes the differentiation of NPC.
**69.** The method of either of embodiments 67 or 68, wherein the MSC are obtained from a human.
**70.** The method of either of embodiments 67 or 68, wherein the solid substrate is selected from the group consisting of plastic, nitrocellulose and glass.
**71.** The method of either of embodiments 67 or 68, wherein the embryonic cortical cells are obtained from a mouse or a rat.
**72.** The method of either of embodiments 67 or 68, wherein the substance is a chemical compound or a polypeptide.
**73.** The method of either of embodiments 67 or 68, wherein the substance is a cell or a cell culture. In certain embodiments, the cell is a mesenchymal stem cell. In additional embodiments, the cell is a descendant of a mesenchymal stem cell that has been transfected with a nucleic acid encoding a Notch intracellular domain (a SB623 cell).
**74.** The method of embodiment 73, wherein the neurogenesis is promoted by a protein expressed on the surface of the cell.
**75.** The method of either of embodiments 67 or 68, wherein the substance is a conditioned medium from a cell culture.
**76.** The method of either of embodiments 67 or 68, wherein differentiation of NPC is compared to differentiation of NPC in the absence of the substance.
**77.** The method of either of embodiments 67 or 68, wherein differentiation of NPC is evidenced by neurite outgrowth, or by expression of a marker selected from the group consisting of microtubule-associated protein 2 (MAP2), doublecortin (DCX), beta-tubulin type III (TuJ1), synaptophysin, neuron-specific enolase, glial fibrillary acidic protein (GFAP), glutamine synthetase, the GLAST glutamate transporter, 2', 3'-cyclic nucleotide 3' phosphodiesterase (CNPase), the O1 antigen, the 04 antigen, myelin basic protein, oligodendrocyte transcription factor 1, oligodendrocyte transcription factor 2, oligodendrocyte transcription factor 3, NG2, and myelin-associated glycoprotein.
**78.** A composition comprising a solid substrate with a biological layer deposited thereon, wherein the biological layer is an extracellular matrix deposited by:
   (a) a mesenchymal stem cell (MSC), or
   (b) a MSC that has been transfected with a nucleic acid, wherein the nucleic acid encodes a Notch intracellular domain but does not encode full-length Notch protein.
**79.** The composition of embodiment 78, wherein the MSC are obtained from a human.
**80.** The composition of embodiment 78, wherein the solid substrate is selected from the group consisting of plastic, nitrocellulose and glass.
**81.** The composition of embodiment 78, further comprising embryonic cortical cells.
**82.** The composition of embodiment 81, wherein the embryonic cortical cells are obtained from a mouse or a rat.
**83.** The composition of embodiment 81, further comprising a test substance.
**84.** The composition of embodiment 83, wherein the test substance is a chemical compound or a polypeptide.
**85.** The composition of embodiment 83, wherein the test substance is a cell or a cell culture. In certain embodiments, the cell is a mesenchymal stem cell. In additional embodiments, the cell is a descendant of a mesenchymal stem cell that has been transfected with a nucleic acid encoding aNotch intracellular domain (a SB623 cell).
**86.** The composition of embodiment 83, wherein the test substance is a conditioned medium from a cell culture.
**87.** A use of a kit for determining the effect of a substance on neuropoiesis, neurogenesis, astrocytogenesis, or oligodendrocytogenesis; the kit comprising the composition of any of embodiments 78-86.
**88.** The use of the kit of embodiment 87, the kit further comprising one or more reagents for
   detection of a neuronal or glial marker molecule.
**89.** The use of the kit of embodiment 88, wherein the detection is by immunohistochemistry.
**90.** The use of the kit of embodiment 89, wherein the reagent comprises one or more antibodies.
**91.** The use of the kit of embodiment 90, wherein the one or more antibodies are specific to one or more antigens selected from the group consisting of microtubule-associated protein 2 (MAP2), doublecortin (DCX), beta-tubulin type III (TuJ1), synaptophysin, neuron-specific enolase, glial fibrillary acidic protein (GFAP), Glast, glutamine synthetase, 2', 3'-cyclic nucleotide 3' phosphodiesterase (CNPase), the 01 antigen, the 04 antigen, myelin basic protein, oligodendrocyte transcription factor 1, oligodendrocyte transcription factor 2, oligodendrocyte transcription factor 3, NG2, and myelin-associated glycoprotein.
**92.** The use of the kit of embodiment 88, wherein the detection is by quantitative reverse transcription/polymerase chain reaction (qRT-PCR).
**93.** The use of the kit of embodiment 92, wherein the reagent comprises one or more oligonucleotide primers or oligonucleotide probes.
**94.** The use of the kit of embodiment 93, wherein the one or more oligonucleotide primers or oligonucleotide probes specifically detect a nucleic acid encoding a protein selected from the group consisting of microtubule-associated protein 2 (MAP2), doublecortin (DCX), beta-tubulin type III (TuJ1), synaptophysin, neuron-specific
enolase, glial fibrillary acidic protein (GFAP), Glast, glutamine synthetase, 2', 3'-cyclic nucleotide 3' phosphodiesterase (CNPase), the 01 antigen, the 04 antigen, myelin basic protein, oligodendrocyte transcription factor 1, oligodendrocyte transcription factor 2, oligodendrocyte transcription factor 3, NG2, and myelin-associated glycoprotein.

### BRIEF DESCRIPTION OF THE DRAWINGS

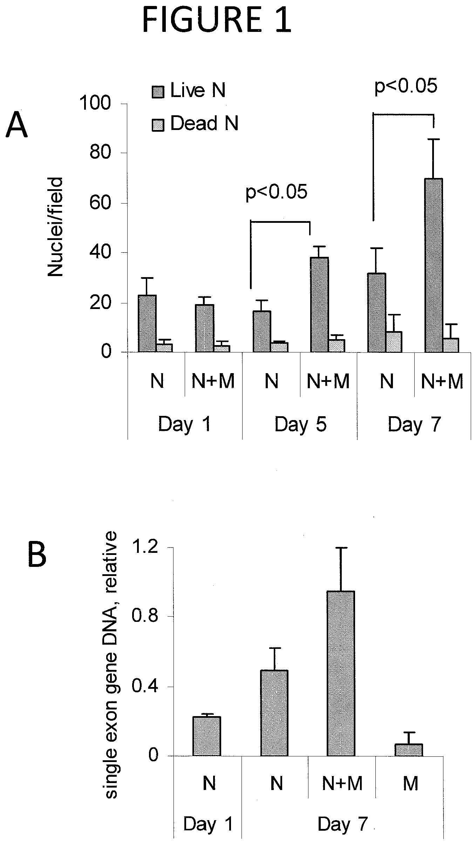

**Figures 1A and 1B** show results of measurements of proliferation of rat neural cells (denoted "N") alone or in co-culture with MSC (denoted "M") on ECM-coated plates. **Figure 1A** shows measurements of the number ofDAPI-stained neural cell (non-MSC) nuclei in co-cultures at three different time points (Day 1, Day 5 and Day 7 after beginning of co-culture). For each pair ofbars, the left-most bar represents the number of live neural cells, and the right-most bar represents the number of dead neural cells, as assessed by nuclear morphology. **Figure 1B** shows cell number, as assayed by relative levels of the rat *noggin* gene, in neural cells (denoted "N") cultured alone or co-cultured with MSC (denoted "M"), and in MSC cultured in the absence of rat neural cells. Data for two time points (Day 1 and Day 7) are shown.

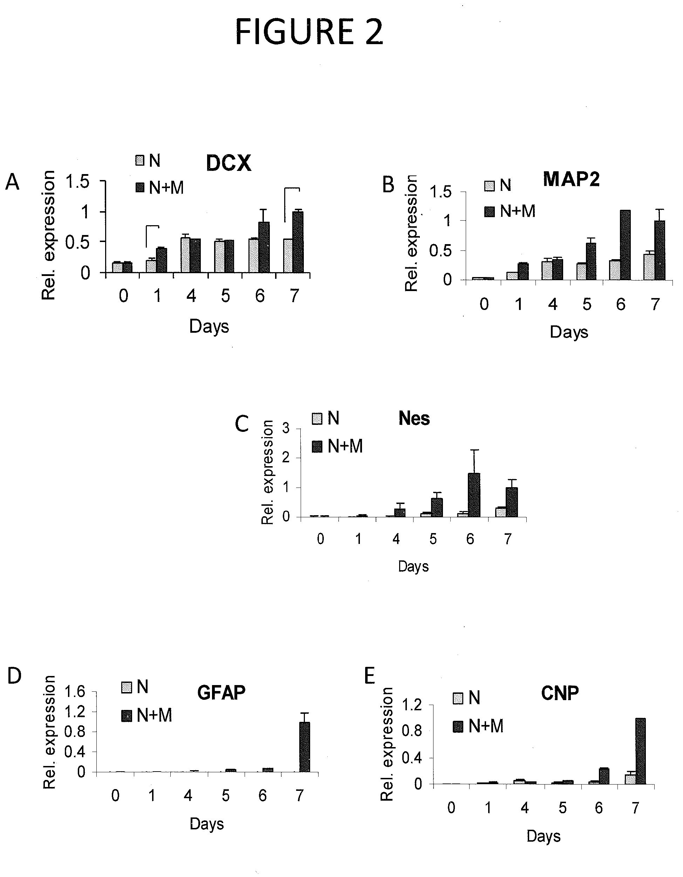

**Figure 2****, panels A to E,** show the time-course of expression of mRNAs for doublecortin (DCX) (FIG. 2A), microtubule-associated protein-2 (MAP2) (FIG. 2B), nestin (Nes) (FIG. 2C), glial fibrillary acidic protein (GFAP) (FIG. 2D) and 2', 3'-cyclic nucleotide 3'phosphodiesterase (CNP) (FIG. 2E) in cultures of rat neural cells (N) and co-cultures of rat neural cells and MSC (N+M) on ECM-coated plates. Co-cultures contained 200 MSC per well. "Days" refers to days after initiation of co-culture.

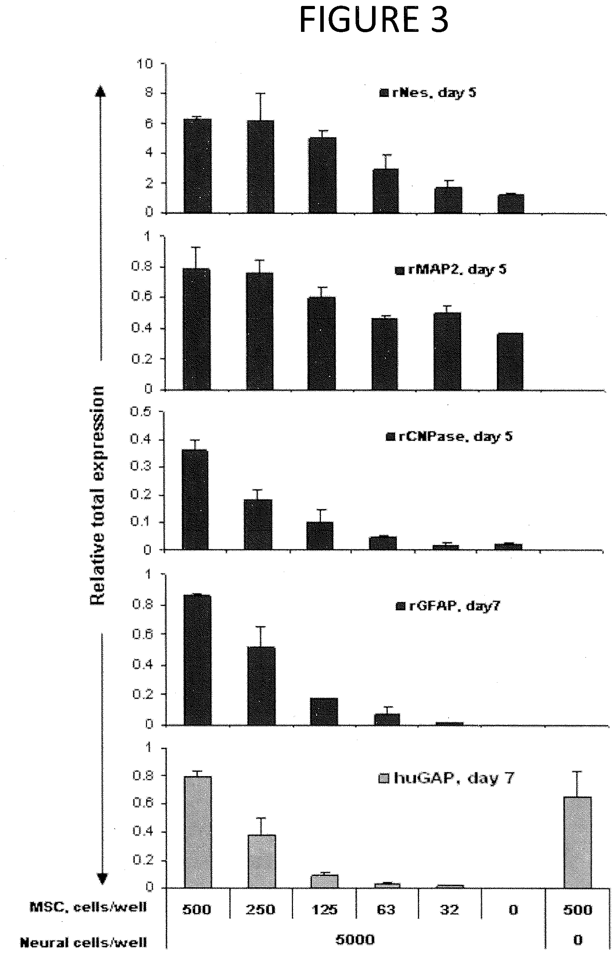

**Figure 3** shows results of quantitative PCR studies indicating that expression ofRNAs encoding various neural markers in rat E18 cortical cells is MSC dose-dependent in co-cultures grown on extracellular matrix (ECM). MSC-dose responses of rat nestin (rNes), MAP2 (rMAP2), and CNPase (rCNPase) gene expression were assessed on day 5, rat GFAP (rGFAP) and human GAP (huGAP) expression were assessed on day 7. No signal from human MCS or SB623 cells alone was detected in any rat expression assays, and no signal from rat cells was detected in the human GAP expression assay.

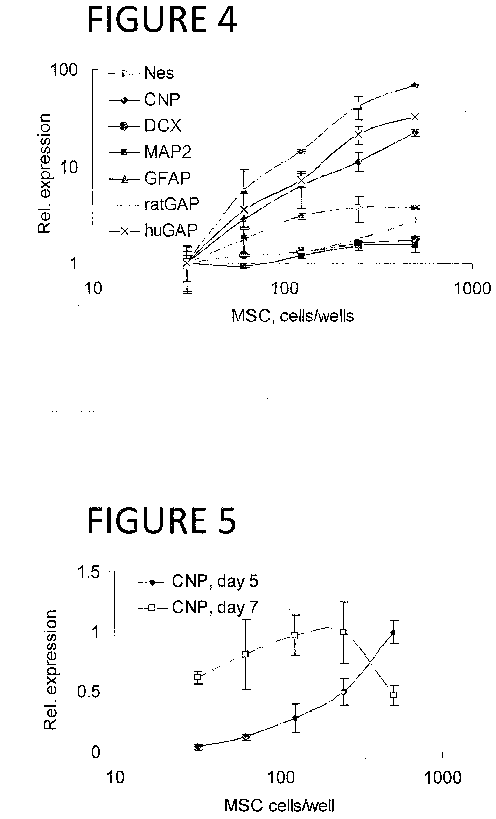

**Figure 4** shows relative expression levels of various markers, determiner by qRT-PCR, in co-cultures of rat neural cells and MSC on ECM-coated plates. The rat markers are nestin (Nes), CNPase (CNP), doublecortin (DCX), microtubule-associated protein-2 (MAP2), glial fibrillary acidic protein (GFAP), and glyceraldehyde-3-phosphate dehydrogenase (ratGAP). The human marker, used to identify and quantitate MSC in the cultures, is glyceraldehyde-3-phosphate dehydrogenase (huGAP). Expression of Nestin and CNPase was assayed after 5 days of co-culture; all other markers were assayed after 7 days of co-culture. An expression level of 1 was arbitrarily assigned to be the level at the lowest MSC dose (32 cells per well).
**Figure 5** shows the effect of MSC concentration on levels of expression of CNPase mRNA at two different stages of co-culture on ECM-coated plates. CNPase mRNA levels were quantitated by qRT-PCR. A relative expression level of 1 was arbitrarily set as the highest level observed on the particular day of assay (day 5 or day 7).

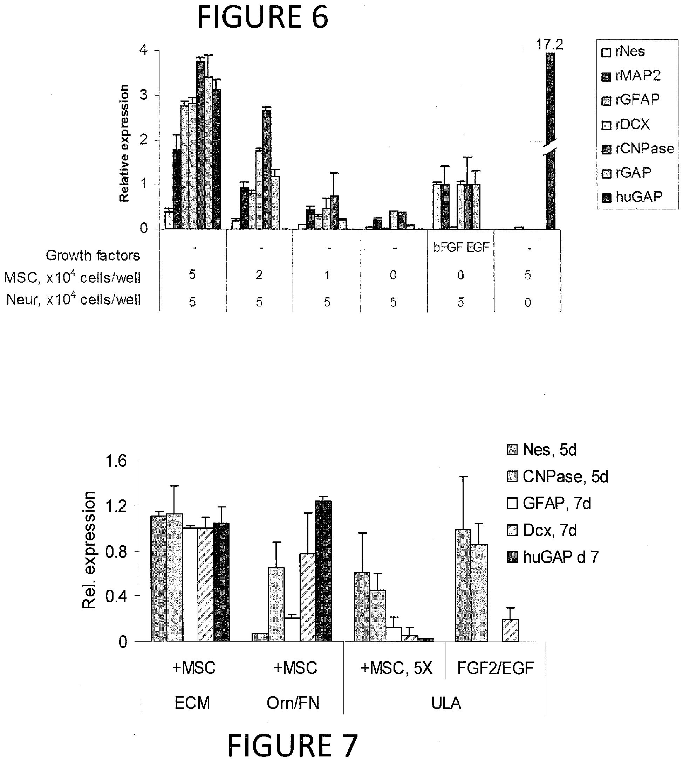

**Figure 6** shows levels of marker expression in co-cultures of rat neural cells and MSC conducted under non-adherent conditions. Neural cells were also cultured without MSC in the presence of bFGF and EGF as a control. For each set of conditions the bars represent, from left to right, expression levels of rat nestin (rNes), rat microtubule-associated protein-2 (rMAP2), rat glial fibrillary acidic protein (rGFAP), rat doublecortin (rDCX), rat 2', 3'-cyclic nucleotide 3' phosphodiesterase (rCNPase), rat glyceraldehyde-3-phosphate dehydrogenase (rGAP) and human glyceraldehyde-3-phosphate dehydrogenase (huGAP). Neural marker gene expression level in the presence of bFGF/EGF was assigned a value of 1 and other values were expressed correspondingly for all markers except GFAP, which was assigned a value of 0.1 in the bFGF/EGF sample.
**Figure 7** shows levels of marker expression in co-cultures of rat neural cells and MSC conducted under different attachment conditions. "ECM" indicates co-culture on plates coated with SB623 cell-derived extracellular matrix. "Orn/FN" indicates co-culture on plates coated with ornithine and fibronectin. "ULA" indicates culture on Ultra Low Attachment plates. On ECM and Orn/FN plates, neural cells were co-cultured with MSC at a 10:1 ratio (1.5 x 10⁴ cells/cm²). On ULA plates, neural cells were cultured either with MSC at a 2:1 ratio ("+MSC, 5X") or in the absence of MSC in medium supplemented with growth factors ("FGF2/EGF"). For each set of conditions, the bars represent, from left to right, expression levels of rat nestin (Nes), rat 2', 3'-cyclic nucleotide 3' phosphodiesterase (CNPase), rat glial fibrillary acidic protein (GFAP), rat doublecortin (DCX), and human glyceraldehyde-3-phosphate dehydrogenase (huGAP). Nestin and CNPase levels were assayed after 5 days of culture or co-culture ("5d"); all other markers were assayed at 7 days ("7d").

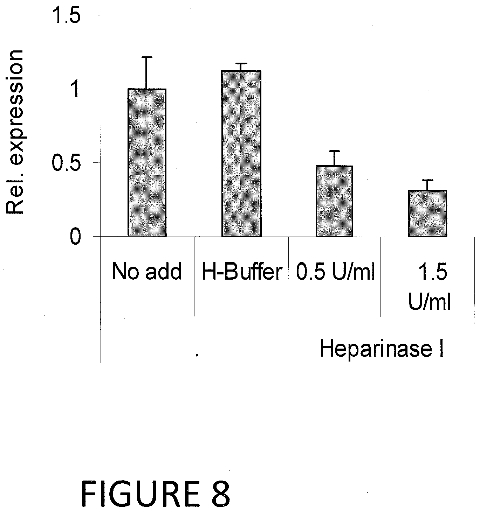

**Figure 8** shows effect of heparinase on expression of nestin mRNA by neural cells. Rat cortical cells were cultured on ECM-coated plates. Prior to plating of the cortical cells, the ECM-coated plates had been treated with two concentrations of heparinase I (0.5 Units/ml and 1.5 Units/ml), or with heparinase buffer ("H-Buffer") or were untreated ("No add"). Nestin mRNA expression was measured by qRT-PCR 5 days after initiation of culture. The amount of nestin mRNA detected in cells cultured on untreated ECM-coated plates was arbitrarily assigned a relative expression level of 1.

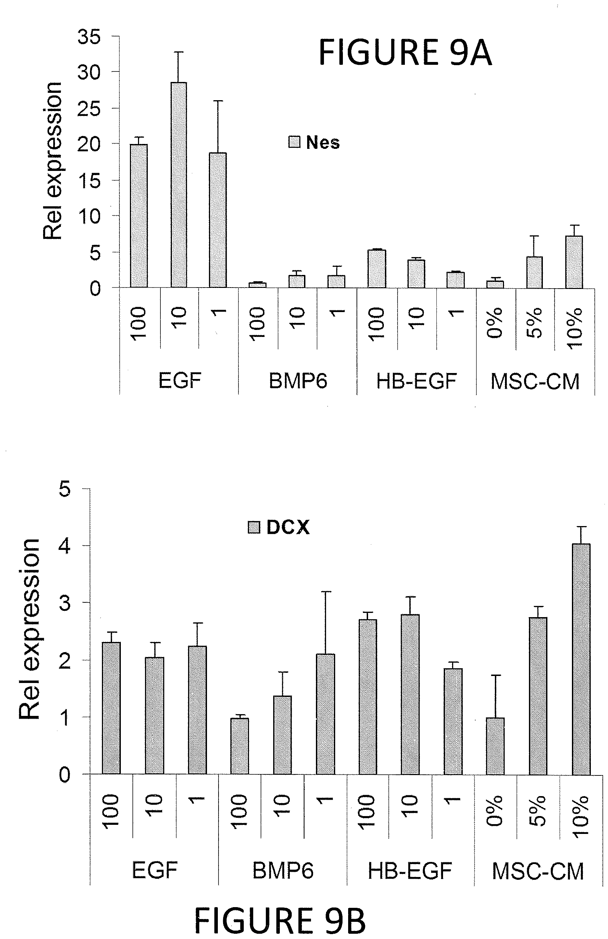

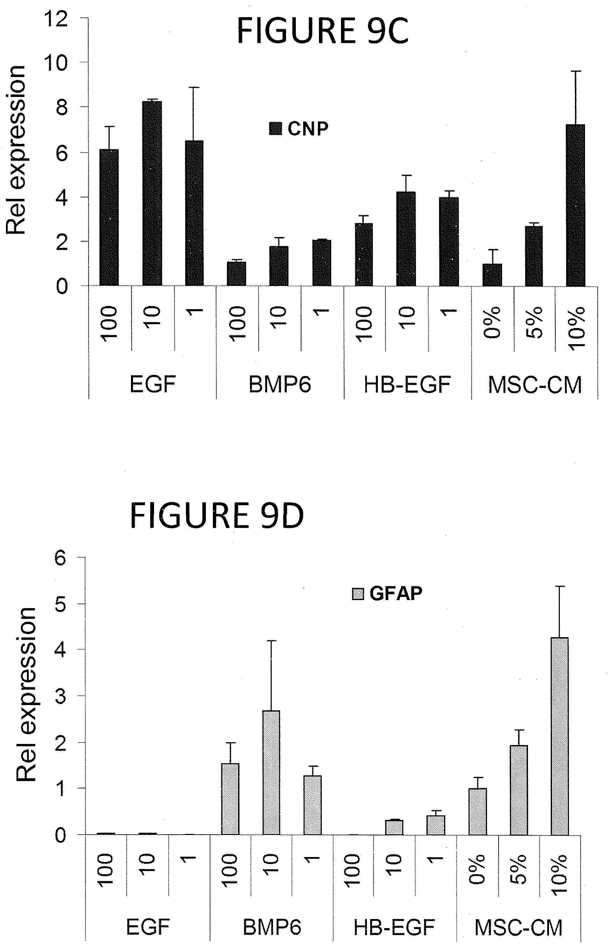

**Figures 9A-9D** show the effects of purified growth factors (EGF, BMP6, HB-EGF) and MSC conditioned medium (CM) on relative expression levels of mRNAs encoding various neural markers, by neural cells cultured on ECM-coated plates, determined by qRT-PCR. **Figure 9A** shows effects on expression of Nestin, a marker for neural precursor cells. **Figure 9B** shows effects on expression of doublecortin (DCX), a marker for nascent neurons. **Figure 9C** shows effects on expression of CNPase, an oligodendrocyte marker. **Figure 9D** shows effects on expression of GFAP, a marker for astrocytes.

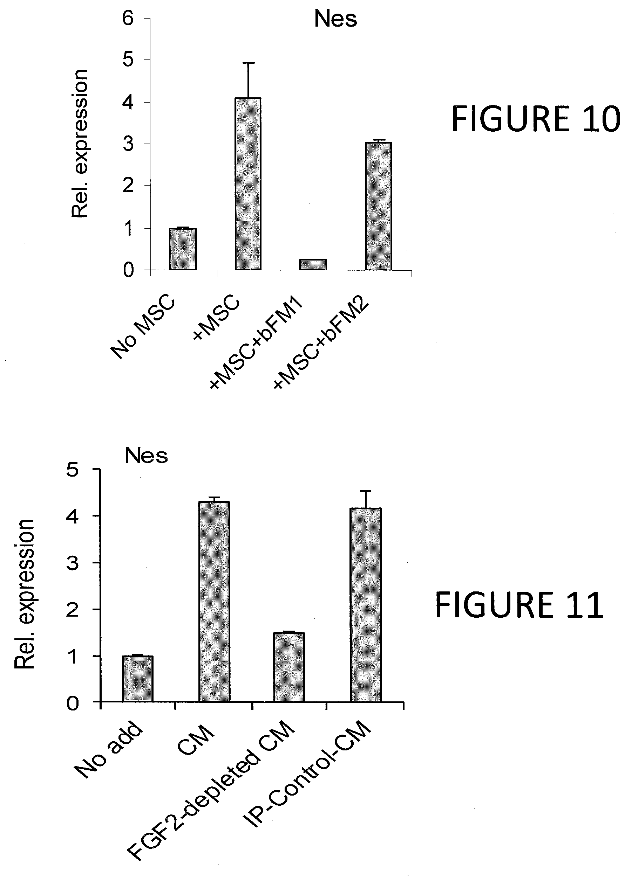

**Figure 10** shows effects of an anti-FGF2 neutralizing antibody on nestin expression by neural cells in neural cell/MSC co-cultures on ECM-coated plates. Rat cortical cells (5,000 cells) were cultured by themselves ("No MSC") or co-cultured with 200 MSC ("+MSC"). Additional co-culture samples also contained either a neutralizing anti-FGF2 antibody ("+MSC+bFM1") or a non-neutralizing anti-FGF2 antibody ("+MSC+bFM2"). Nestin expression was assayed 5days after beginning of culture or co-culture. Levels of nestin expression in cortical cells cultured in the absence of MSC were arbitrarily assigned a relative expression value of 1.
**Figure 11** shows the effects of MSC conditioned medium, and of FGF2-depleted MSC conditioned medium, on nestin expression in cultured rat neural cells. Rat cortical cells were cultured on ECM-coated plates without further additions ("No add"), with MSC conditioned medium ("CM"), with MSC conditioned medium that had been depleted of FGF2 by immunoprecipitation ("FGF2-depleted CM"), and with MSC conditioned medium treated with a control antibody that did not react with FGF2 ("IP-Control-CM"). Nestin expression was assayed 5days after beginning of culture. Levels of nestin expression in cortical cells cultured in the absence of conditioned medium were arbitrarily assigned a relative expression value of 1.

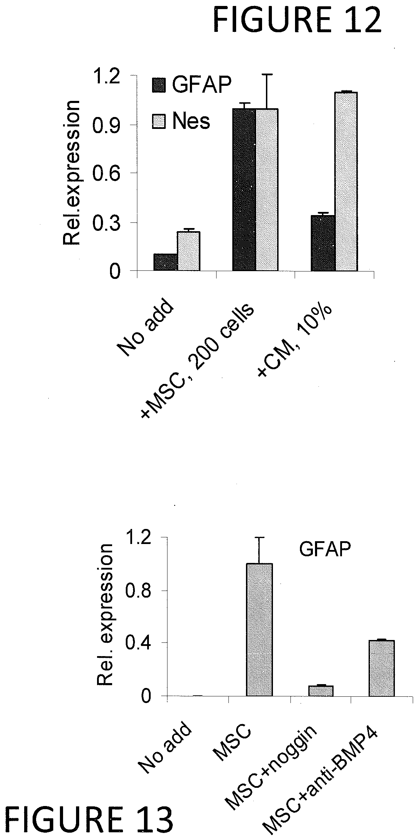

**Figure 12** shows levels of mRNAs encoding nestin (Nes) and glial fibrillary acidic protein (GFAP), expressed by neural cells cultured on ECM-coated plates in the presence of 200 mesenchymal stem cells ("+MSC, 200 cells") or a 1:10 dilution of conditioned medium from mesenchymal stem cells ("+CM, 10%"). Control cells were cultured in the absence of MSC or conditioned medium ("No add"). Assay for nestin was conducted 5 days after beginning of culture; assay for GFAP was conducted 7 days after beginning of culture. The level of each marker expressed in co-culture with MSC was arbitrarily assigned a relative expression value of 1.
**Figure 13** shows levels of GFAP mRNA, assayed 7 days after beginning of culture or co-culture, in rat cortical cells cultured on ECM-coated plates. Cortical cells were co-cultured with MSC ("MSC"), co-cultured with MSC in the presence of 30 ng/ml recombinant noggin protein ("MSC+noggin"), or co-cultured with MSC in the presence of an anti-BMP4 antibody ("MSC+anti-BMP4"). The level of GFAP mRNA expressed in co-culture with MSC was arbitrarily assigned a relative expression value of 1.

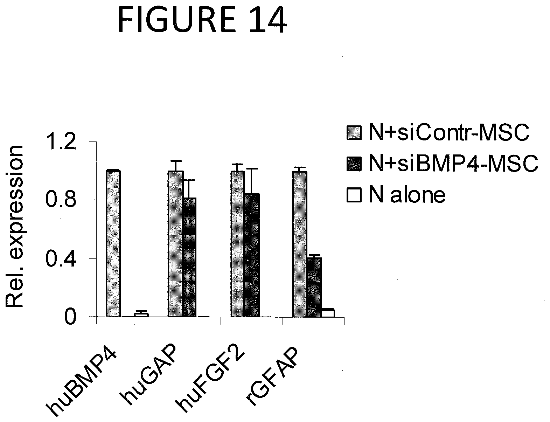

**Figure 14** shows expression levels of mRNAs for human bone morphogenetic protein-4 ("huBMP4"), human glyceraldehyde-3-phosphate dehydrogenase ("huGAP"), human fibroblast growth factor-2 ("huFGF2") and rat glial fibrillary acidic protein ("rGFAP") in co-cultures of rat neural cells and MSC on ECM-coated plates. Prior to co-culture, MSC were transfected with siRNA pools targeted to human BMP-4 sequences ("N+siBMP4-MSC") or a control non-BMP4-targeted siRNA ("N+siContr-MSC"). Neural cells were also cultured separately in the absence of MSC ("N alone").

### DETAILED DESCRIPTION

It has proven difficult to establish *in vitro* culture conditions that will support the growth and differentiation of the various different types of neural cells. The present inventors have devised an *in vitro* use of a culture system in which neural
precursor cells, neurons, astrocytes and oligodendrocytes are all able to grow and differentiate. The use of the culture system disclosed herein thus allows, for the first time, quantitative evaluation of the effect of a test substance on the growth and differentiation of neural cells. The system comprises a culture of neural cells *(e.g.* embryonic rodent cortical cells) on an extracellular matrix in the presence of a test substance, followed by analysis of the neural cell culture for the expression of one or more marker molecules. The extracellular matrix used in these assays is produced by (a) a mesenchymal stem cell, or (b) a mesenchymal stem cell that has been transfected with a nucleic acid, wherein the nucleic acid encodes a Notch intracellular domain but does not encode full-length Notch protein *(e.g.,* a SB623 cell).

Various aspects of this system contribute to its ability to provide quantitative information on the potency of various neurogenic and gliogenic factors. In one aspect, the neural cells are cultured on an extracellular matrix produced by MSC or SB623 cells (cells that have been derived from MSC by transfecting MSC with a vector containing sequences encoding an Notch intracellular domain). In another aspect, the amount of time that the neural cells are co-cultured with a test substance is chosen to optimize detection and quantitation of the marker that is being assayed. The duration of co-culture prior to assay is unique to each marker. For example, co-culture is conducted for five days for measurement of nestin and CNPase; and for seven days for measurement of GFAP, DCX and MAP2. In yet another aspect, the concentration of cells in the culture is optimized. For example, neural cells are used at a concentration of 1.5 x 10⁴ cells/ml; MSC and SB623 cells are used at a concentration of 0.5-1.5 x 10³ cells/mi.

The quantitative assay system disclosed herein utilizes ECM from mesenchymal cells such as MSC and their derivatives *(e.g.,* SB623 cells) as a biological substrate for co-cultures oftest substances *(e.g.,* MSC or their derivative SB623 cells, conditioned medium, growth factors, cytokines) and neural cell populations, and provides a culture system that is favorable to the growth of both mesenchymal cells and neural cells. Such a system, in turn, allows quantitation of the effects of mesenchymal cells, as well as effects of other cells and substances, on the growth and differentiation of various types of neural cells.

The advantages of the assays described herein include that fact that developmental transitions occur under physiological conditions and over a physiological time-course, rather than in response to abnormal physical conditions, such as attachment or aggregation (*cf.* neurosphere cultures). In addition, the stage of development of the cells being assayed can be easily determined, as development does not occur in the interior of a neurosphere. Finally, the assays disclosed herein do not require external growth factors; thus allowing the effects of such factors to be quantitated in this system.

Using this system, the inventors have determined that not only does mesenchymal cell ECM support the growth of neural cell populations (such as, for example, embryonic cortical cells), but that addition of MSC or SB623 cells to neural cell populations growing on mesenchymal cell ECM substantially enhances growth and differentiation of all neural lineages (*e.g.,* neurons, astrocytes and oligodendrocytes).

Compared to existing co-culture systems, much lower ratios of mesenchymal cells to neural cells are capable of inducing significant growth and differentiation of neural cells in the ECM-based co-cultures described herein. For example, the assay systems described herein are sensitive enough to detect the effect of approximately 50 mesenchymal cell on 5,000 neural cells.

Provided herein are quantitative assays for neurogenic and gliogenic factors, as well as factors that promote the growth and differentiation of neural precursor cells. The assays can also be used to identify and quantitate sources of such factors, such as cell cultures or conditioned media.

To conduct the assays, MSC or SB623 cells (referred to collectively as "mesenchymal cells") are grown in a vessel, such as a tissue culture dish, for a period of time sufficient for the cells to lay down an extracellular matrix on the surface of the vessel. Any solid substrate can be used as a surface on which the cells are grown, as long as it supports the growth of the cells and the elaboration of an extracellular matrix by the cells. Suitable substrates include plastic, glass or nitrocellulose. Further, the substrate may be coated with a substance such as, for example, fibronectin or collagen, or a reconstituted basement membrane such as, for example, Matrigel™.

An example of a suitable substrate is a plastic tissue culture dish or flask. The cells can be grown for one day, two days, three days, one week, two weeks, one month, or any time interval therebetween as desired. For additional details on ECM elaborated by MSC and SB623 cells, see U.S. Patent Application Publication No. 2010/0310529, which describes ECM elaborated by MSC and SB623 cells (denoted "differentiation-restricted descendants of MASCs" in that publication) and its properties.

MSC can be obtained by selecting adherent cells from bone marrow samples. Bone marrow can be obtained commercially (*e.g.,* from Lonza, Walkersville, MD) or from bone marrow biopsies. Other sources of mesenchymal stem cells include, for example, adipose tissue, dental pulp, cord blood, placenta and the decidua. MSC can be obtained from any animal, including mammals, and including humans.

Exemplary disclosures of MSC are provided in U.S. patent application publication No. 2003/0003090; Prockop (1997) Science 276:71-74 and Jiang (2002) Nature 418:41-49. Methods for the isolation and purification of MSC can be found, for example, in U.S. Patent No. 5,486,359; Pittenger et al. (1999) Science 284:143-147 and Dezawa et al. (2001) Eur. J. Neurosci. 14:1771-1776. Human MSC are commercially available (*e.g.,* BioWhittaker, Walkersville, MD) or can be obtained from donors by, *e.g.,* bone marrow aspiration, followed by selection for adherent bone marrow cells. See, *e.g.,* WO 2005/100552.

SB623 cells are derived from MSC by transfecting MSC with a vector containing sequences that encode a Notch intracellular domain (NICD) but do not encode the full-length Notch protein, such that the transfected cells express exogenous NICD but do not express exogenous full-length Notch protein. Methods for obtaining MSC, and for deriving SB623 cells from MSC populations, are described, for example, in US Patent No. 7,682,825 and in US Patent Application Publication No. 2010/0266554, the disclosures of which are incorporated by reference for the purposes of describing MSC and SB623 cells, and methods of obtaining these cells.

Subsequent to growth on the substrate for a predetermined amount of time, the MSC or SB623 cells are removed from the substrate, leaving behind an extracellular matrix deposited on the substrate. Methods of removing cells from a substrate are well known in the art. In the practice of the methods disclosed herein, removal of cells from the substrate must be sufficiently gentle that the ECM that has been elaborated by the cells remains on the substrate. Such methods include, for example, treatment with non-ionic detergent (*e.g.,* Triton X-100, NP40) and alkali (*e.g.* NH₄OH). See the "Examples" section *infra* for additional details.

The ECM-containing substrate is then used as a substrate for co-culture of neural cells and one or more test substance(s), and the effect of the test substance(s) on the neural cells is determined and quantitated. Introduction of the neural cells and the test substance to the culture can be simultaneous, or in either order.

Any type of neural cell or neural cell population can be used; such cells are known in the art. A convenient source of neural cell populations are rodent embryonic cortical cells (*e.g.,* from rat or mouse), which can be obtained commercially (BrainBits, Springfield, IL). In certain embodiments, the neural cell population is enriched in neural precursor cells.

A test substance can be any chemical compound, macromolecule (*e.g.,* nucleic acid or polypeptide), cell, cell culture, cell fraction or tissue, or combination thereof. For example, growth factors and cytokines, low molecular weight organic compounds, mRNA molecules, siRNA molecules, shRNA molecules, antisense RNA molecules, ribozymes, DNA molecules, DNA or RNA analogues, proteins (*e.g.,* transcriptional regulatory proteins), antibodies (*e.g.,* neutralizing antibodies), enzymes (*e.g.,* nucleases), glycoproteins, glycans, proteoglycans, cells, cell membrane preparations, cell cultures, conditioned medium from cell cultures, subcellular fractions and tissue slices or tissue fractions are all suitable test substances. Test substances can also include electromagnetic radiation such as, for example, X-rays, light (*e.g.,* ultraviolet, infrared) or sound (*e.g.,* subsonic or ultrasonic radiation). In certain embodiments, the combination of a protein and a neutralizing antibody to the protein is used as a test substance.

Naturally-occurring test substances can include soluble molecules (*e.g.,* proteins) synthesized and secreted by cells, as well as molecules (*e.g.,* proteins) that are synthesized by a cell, transported to the cell surface, and remain embedded in the cell surface, with all or a portion of the molecule exposed to the exterior of the cell (*i.e.,* surface molecules, surface proteins or surface glycoproteins).

Neural cells and test substances are co-cultured for an appropriate amount of time, as determined by the practitioner of the method. For example, co-culture can be conducted for 1 hour, two hours, three hours, four hours, six hours, 12 hours, one day, two days, three days, four days, five days, six days, one week, two weeks, one month, or any time interval therebetween.

The effect(s) of the test substance(s) on the neural cells is determined by measuring the expression of one or more markers in the neural cells. Depending on the marker or markers chosen, it is possible to assay for formation of neural precursor cells, neurons, astrocytes, or oligodendrocytes.

In one embodiment, the effect of a particular protein, either native or recombinant, on neurogenesis or gliogenesis can be determined by adding the protein to a culture of neural cells growing on a MSC or SB623 ECM and assaying for the appropriate neuronal or glial marker. Optionally, a low concentration (1%, 2%, 5%, 10%, 20%, 30%, 40%, 50% or any value therebetween) of conditioned medium from MSC or SB623 cells can also be included in the culture. For example, inclusion of conditioned medium can provide additional factors required for the process under study, other than the one being tested, thereby allowing the effect of one component of a multi-factor signaling system to be assessed.

Molecular and morphogenetic markers for neural precursor cells, neurons, astrocytes and oligodendrocytes are well-known in the art; the following are provided as examples.

Markers for neural precursor cells include, for example, nestin, glutamate transporter (GLAST), 3-phosphoglycerate dehydrogenase (3-PGDH, astrocyte precursors), ephrin B2 (EfnB2), Sox2, Pax6, and musashi. In certain embodiments, proliferative capacity can also be used as a marker for neural precursor cells. Proliferative capacity can be measured, for example, by incorporation of bromodeoxyuridine, carboxyfluorescein diacetate succinimidyl ester (CFSE) labeling, expression of Ki-67 or expression of proliferating cell nuclear antigen (PCNA).

Markers for neurons include, for example, microtubule-associated protein 2 (MAP2), β-tubulin isotype III (also known as β-III tubulin and TuJ-1), doublecortin (DCX), neurofilament proteins (*e.g.,* neurofilament-M), synaptophysin, and neuron-specific enolase (also known as enolase-2 and gamma enolase). Neurite outgrowth can also be used as a marker for neuronal development.

Additional neuronal markers are listed in the following table:

| Early Neuronal Markers | |
|---|---|
| ATH1 [MATH1] | Nuclear |
| ASH1 [MASH1] | Nuclear |
| Hes5 | Nuclear |
| HuC (Hu, Rodent) | Very early marker, Nuclear |
| HuD | Nuclear |
| Internexin α | Cytoplasmic, soma, early neurites |
| L1 neural adhesion molecule | Plasma membrane |
| MAP1 B [MAP5] | Cytoplasmic, soma, dendritic |
| MAP2A, 2B | Cytoplasmic, soma, dendritic |
| Nerve Growth Factor Rec (NGFR) p75 | Plasma membrane |
| Nestin | Cytoplasmic |
| NeuroD | Nuclear |
| Neurofilament L 68 kDa | Cytoplasmic |
| Neuron Specific Enolase (NSE) | Cytoplasmic |
| NeuN | Nuclear, |
| Nkx-2.2 [NK-2] | Nuclear |
| Noggin | Secreted |
| Pax-6 | Nuclear, eye development |
| PSA-NCAM, clone 2-2B | Plasma membrane |
| Tbr1 | Nucleus |
| Tbr2 | Nucleus |
| Tubulin, βIII | Cytoplasmic, neuritis |
| TUC-4 | Axonal growth cones |
| Tyrosine Hydroxylase (TH) | Cytoplasmic, adrenergic neuron lineage |

| Immature Neuron & Growth Cone Markers | |
|---|---|
| Collapsin Response Mediated Protein 1 [CRMP1] | Growth cone |
| Collapsin Response Mediated Protein 2 [CRMP2] | Growth cone |
| Collapsin Response Mediated Protein 5 [CRMP5] | Growth cone |
| Contactin-1 | Cytoplasmic |
| Contactin-1 | Cytoplasmic |
| Cysteine-rich motor neuron 1 [CRIM1] | Cytoplasmic, motor neurons |
| c-Ret phosphor Serine 696 | Cytoplasmic |
| Doublecortin [DCX] | Cytoplasmic, migrating neurons |
| Ephrin A2 | Plasma membrane |
| Ephrin A4 | Plasma membrane |
| Ephrin A5 | Plasma membrane |
| Ephrin B1 | Plasma membrane |
| Ephrin B2 | Plasma membrane |
| Ephrin B phosphoTyr298 | Plasma membrane |
| Ephrin B phosphoTyr317 | Plasma membrane |
| Ephrin B phosphoTyr331 | Plasma membrane |
| GAP-43 | Plasma membrane |
| GAP-43, phosphoSer 41 | Plasma membrane |
| HuC/D | |
| Internexin alpha | |
| Laminin-1 | Plasma membrane |
| LINGO-1 | Cytoplasmic |
| MAP1B [MAP5] | |
| Mical-3 | Growth cones |
| NAP-22 | Plasma membrane, growth cones |
| NGFR | |
| Nestin | |
| Netrin-1 | Plasma membrane |
| Neurite Outgrowth Quantification Assay kit | |
| Neuropilin | Plasma membrane |
| Plexin-A1 | Plasma membrane, growth cone |
| RanBPM | Cytoplasmic, growth cone |
| Semaphorin 3A | Plasma membrane, growth cone |
| Semaphorin 3F | Plasma membrane |
| Semaphorin 4D | Plasma membrane |
| Slit2 | Secreted |
| Slit3 | Secreted |
| Staufen | Cytoplasmic |
| Tbr 1 & 2 | |
| Trk A | Plasma membrane |
| Tubulin, βIII | |
| TUC-4 | |

| Neuronal Markers - Nuclear | |
|---|---|
| HuD | Postmitotic neurons |
| NeuN | Nuclei of most neurons |
| Peripherin | Peripheral neurons |

| Neuronal Markers - Cytoplasmic | |
|---|---|
| MAP2A, B, C. | All neurons, soma, dendrites |
| Tubulin, βIII | All neurons, soma, axons |
| CDK5 [NCLK], perikarya | Soma |
| MacMARCKS | Soma |
| MARCKS | Soma |
| Neurofilaments | All neurons, soma, axons, proximal dendrites |
| Neuron Specific Enolase (NSE) | Cytoplasmic |
| Parvalbumin | Neurons, muscle |
| Protein Gene Product 9.5 [PGP9.5] | All neurons, neuroendocrine cells |
| STEP | NMDAR expressing neurons |
| STOP [N-STOP, Stable tubule-only polypeptide] | Soma, dendrites |
| Tau | Axons |
| Tau phospho specific | Axons |
| CD90 [Thy-1] | Neurons, thymocytes, connective tissue |
| CDw90 [Thy-1.1] | Neurons, thymocytes, connective tissue |
| Encephalopsin | PO, PVN, Purkinje cells, other select regions |
| GAD65 [Glutamate Decarboxylase] | Glutamatergic neurons |
| GAP-43 [Growth Associated Protein 43] | Differentiating and regenerating neurons |
| LINGO-1 | Differentiating and regenerating neurons |
| Na+/K+ ATPase subunits | All neurons |
| Neuron Cell Surface Antigen [A2B5] | Neurons, glia |
| Post-synaptic receptors | |
| 4.1G | Neuron specific |
| Acetylcholinesterase | Cholinergic |
| Ack1 | Clathrin-mediated endocytosis |
| AMPA Receptor Binding Protein [ABP] | Postsynaptic |
| ARG3.1 | Presynaptic, plasticity related |
| Arp2 | Most neurons |
| E-Cadherin | Cell junctions |
| N-Cadherin | Cell junctions |
| Calcyon | Postsynaptic, Dopaminergic |
| Catenin, alpha and beta | Cell junctions |
| Caveolin | Presynaptic |
| CHAPSYN-110 [PSD93] | Postsynaptic |
| Chromogranin A | Peripheral, Neuroendocrine, presynaptic |
| Clathrin light chain | Presynaptic |
| Cofilin | Postsynaptic |
| Complexin 1 [CPLX1, Synaphin 2] | Presynaptic |
| Contactin-1 | Cell junctions |
| CRIPT | Postsynaptic |
| Cysteine String Protein [CSP] | Presynaptic |
| Dynamin 1 and 2 | Presynaptic |
| Flotillin-1 | Presynaptic |
| Fodrin | Perisynaptic |
| GRASP | Postsynaptic |
| GRIP1 | Postsynaptic |
| Homer | Postsynaptic |
| Mint-1 | Presynaptic |
| Munc-18 | Presynaptic |
| NSF | Presynaptic |
| PICK1 | Postsynaptic |
| PSD-95 | Postsynaptic |
| RAB4 | Presynaptic |
| Rabphillin 3A | Presynaptic |
| SAD A & B | Presynaptic |
| SAP-102 | Postsynaptic |
| SHANK1a | Postsynaptic |
| SNAP-25 | Presynaptic |
| Snapin | Presynaptic |
| Spinophilin [Neurabin-1] | Postsynaptic, dendritic spines |
| Stargazin | Postsynaptic, AMPAR |
| Striatin | Postsynaptic, dendritic |
| SYG-1 | Perisynaptic |
| Synaptic Vesicle Protein 2A & 2B | Presynaptic |
| Synapsin 1 | Presynaptic |
| Synapsin 1 phospho specific | Presynaptic |
| Synaptobrevin [VAMP] | Presynaptic |
| Synaptojanin 1 | Presynaptic |
| Synaptophysin | Presynaptic |
| Synaptotagmin | Presynaptic |
| Synaptotagmin phospho specific | Presynaptic |
| synGAP | Postsynaptic |
| Synphilin-1 | Perisynaptic, synuclein related |
| Syntaxin 1, 2, 3, 4 | Presynaptic |
| Synuclein alpha | Presynaptic |
| VAMP-2 | Presynaptic |
| Vesicular Acetylcholine Transporter [VAChT] | Presynaptic |
| Vesicular GABA transporter [VGAT; VIAAT] | Presynaptic |
| Vesicular Glutamate Transporter 1, 2, 3 [VGLUT] | Presynaptic |
| Vesicular Monoamine Transporter 1, 2 [VMAT] | Presynaptic |

| Neuronal Markers - Cholinergic | |
|---|---|
| Acetylcholine (ACh) | Presynaptic |
| Acetylcholinesterase | Perisynaptic |
| Choline Acetyltransferase [ChAT] | Cytoplasmic |
| Choline transporter | Plasma Membrane |
| Vesicular Acetylcholine Transporter [VAChT] | Presynaptic |

| Neuronal Markers - Dopaminergic | |
|---|---|
| Adrenaline | Presynaptic |
| Dopamine | Presynaptic |
| Dopamine Beta Hydroxylase [DBH] | Cytoplasmic |
| Dopamine | Plasma |
| Transporter [DAT] | Membrane |
| L-DOPA | Cytoplasmic |
| Nitric Oxide-Dopamine | Presynaptic |
| Norepinephrine | Presynaptic |
| Norepinephrine Transporter [NET] | Plasma Membrane |
| Parkin | Cytoplasmic |
| Tyrosine Hydroxylase [TH] | |
| TorsinA | Cytoplasmic, ER |

| Neuronal Markers - Serotonergic | |
|---|---|
| DL-5-Hydroxytryptophan | Presynaptic |
| Serotonin | Presynaptic |
| Serotonin Transporter [SERT] | Plasma Membrane |
| Tryptophan Hydroxylase | Cytoplasmic |

| Neuronal Markers - GABAergic | |
|---|---|
| DARPP-32 | GABAergic neurons in CNS; Medium spiny neurons |
| GABA | Presynaptic |
| GABA Transporters 1, 2, 3 | Plasma Membrane |
| Glutamate Decarboxylase [GAD] | Cytoplasmic |
| Vesicular GABA transporter [VGAT; VIAAT] | Presynaptic |

| Neuronal Markers - Glutamatergic | |
|---|---|
| Glutamate | Presynaptic |
| Glutamate Transporter, Glial | Plasma Membrane |
| Glutamate Transporter, Neuronal | Plasma Membrane |
| Glutamine | Cytoplasmic |
| Glutamine Synthetase, clone Gs-6 | Cytoplasmic |
| Vesicular Glutamate Transporter 1, 2, 3 [VGLUT] | Presynaptic |

Glial fibrillary acidic protein (GFAP), glutamate transporter (GLAST), 3-PGDH and glutamine synthetase can be used as markers for astrocytes.

Markers for oligodendrocytes include, for example, the A2B5 antigen, galactocerebroside (GalC), 2', 3'-cyclic nucleotide 3' phosphodiesterase (CNPase), the O1 antigen, the 04 antigen, myelin basic protein, oligodendrocyte transcription factor 1, oligodendrocyte transcription factor 2, oligodendrocyte transcription factor 3, NG2, and myelin-associated glycoprotein.

Expression of markers can be measured by techniques that are well-known in the art. For example, expression of proteins can be measured and quantitated by immunofluorescence, immunohistochemistry (IHC), ELISA and protein blotting (*e.g.,* Western blots).

Expression of mRNA can be measured and quantitated by methods including, for example, blotting, nuclease protection and reverse transcription-polymerase chain reaction (RT-PCR).

Depending on the test substance and marker being assayed, it may be necessary to ensure that the assay is specific for the molecule produced by the neural cell and does not cross-react with the same or a similar molecule produced by the test substance, especially if the test substance is a cell, such as a MSC or a SB623 cell. For example, MSC express nestin; therefore, ifnestin is being assayed as a marker for a neural precursor cell in a co-culture of rat cortical cells and human MSC, an antibody specific for rat nestin is used in the assay. Similarly, if nucleic acid expression is assayed, such as by quantitative reverse transcription/polymerase chain reaction (qRT-PCR) or TaqMan, species-specific primers (and probes, if applicable) are used.

In certain embodiments, the expression of a marker by a neural cell in the assay described above *(i.e.* co-culture of neural cells and test substance) is compared to the expression of the same marker in neural cells in the absence of the test substance(s).

The assays described herein can also be used to quantitate the differentiation of neural precursor cells (NPCs) by measuring expression of markers characteristic of the progeny of NPCs, which include neurons, astrocytes and oligodendrocytes. Such markers are well-known in the art and exemplary markers have been described herein.

In certain embodiments, a use of a kit for assaying the neurogenic or gliogenic potential of a test substance, or for assaying the ability of a substance to promote the
growth and/or differentiation of neuronal precursor cells, is provided. The kit contains one or both of MSC and SB623 cells (optionally in a cryopreserved state), along with one or more culture vessels, and optionally culture medium, to allow the user to grow the MSC or SB623 cells on the culture vessel. The kit may also contain reagents *(e.g.*, nonionic detergents such as Triton X-100 or Nonidet P-40; ammonium hydroxide) for removing the MSC or SB623 cells from the culture vessel so as to leave an extracellular matrix deposited on the surface of the culture vessel. The kit can also contain a sample of neural cells *(e.g.,* rat E18 cortical cells). Labeled antibodies to various neuronal and glial markers may also be included in the kit; and oligonucleotide probes and/or primers specific for mRNAs encoding neuronal and glial markers can also be included. Any type of reagent that will detect a neuronal or glial marker *(e.g.,* a protein) or its encoding mRNA can be included in the kit. Reagents and/or buffers and/or apparatus suitable for immunohistochemistry, FACS, RT-PCR, electrophysiology and pharmacology can also be included in the kit.

In additional embodiments, a kit for use as disclosed herein can contain one or more culture vessels with an ECM from MSC or SB623 cells deposited thereon. Such a kit can optionally include neural cells and reagents *(e.g*., antibodies, probes, primers) to detect neuronal and/or glial markers. Such a kit can also optionally include reagents and/or buffers suitable for immunohistochemistry, FACS, RT-PCR, electrophysiology and pharmacology.

In additional embodiments, a kit can contain purified extracellular matrix from MSC or SB623 cells (or a mixture thereof), for application to a culture vessel.

In further embodiments, a kit comprises a solid substrate *(e.g.,* a culture vessel) with a biological layer deposited thereon, wherein the biological layer is an extracellular matrix deposited by:
(a) a mesenchymal stem cell, or
(b) a mesenchymal stem cell that has been transfected with a nucleic acid, wherein the nucleic acid encodes a Notch intracellular domain but does not encode full-length Notch protein.

In the operation of the kits, neural cells are grown in contact with an extracellular matrix from MSC or SB623 cells, in the presence of a test substance, and the neural cells are analyzed for the expression of a chosen neuronal or glial marker. For use of certain of the aforementioned kits, deposition of the ECM on a culture vessel (by MSC and/or SB623 cells) and removal of the cells that elaborated the ECM, is conducted by the user prior to adding the neural cells and the test substance to the culture vessel.

### EXAMPLES

### General Methods

### MSC and SB623 cell preparation

MSC and SB623 cell preparation has been described [29]. Briefly, human adult bone marrow aspirates (Lonza, Walkersville, MD) were grown in aMEM (Mediatech, Herndon, VA) supplemented with 10% fetal bovine serum (FBS) (Hyclone, Logan, UT), 2mM L-glutamine, and penicillin/streptomycin (both from Invitrogen, Carlsbad, CA). On the second passage, some cells were cryopreserved (MSC preparation) and some cells were plated for the preparation of SB623 cells. For SB623 cell preparation, MSC were transfected with a pCI-neo expression plasmid encoding the human Notch1 intracellular domain (NICD). After one day of culture, transfected cells were placed under selection with G418 (Invitrogen) for 7 days, after which selection was removed and the cultures were grown and expanded by passaging twice. SB623 cells were then harvested and cryopreserved using Cryostor CS5 (BioLife Solutions, Bothell, WA). Cells from 3 different donors were used in the studies described herein. MSC and SB623 cells were thawed and washed once with αMEM before use. For co-culture experiments, cells were then resuspended in a neural growth medium consisting of Neurobasal medium supplemented with 2% B27 and 0.5 mM GlutaMAX (all from Invitrogen). For the production of ECM coating or the production of conditioned medium (CM), cells were plated in αMEM supplemented with 10% FBS and penicillin/streptomycin.

### Plate coating

For the preparation of wells coated with ECM, SB623 cells were plated at 3x10⁴cells/cm² in 96-well plates or on glass cover slips (Fisher Scientific, Pittsburgh, PA) which were placed into 12-well plates (all plates were purchased from Corning Inc, Corning, NY) and grown for 5 days. Subsequently the medium was changed to serum-free, and the cells were cultured for an additional 2 days. Cells were then removed from the ECM using a protocol described previously [29] with some modifications. Briefly, cells were treated with 0.2% Triton X-100 (Sigma-Aldrich, St. Louis, MO) in water at room temperature for 40 min; then cell lysates were carefully aspirated, and a 1:100 (v/v) solution of concentrated NH₄OH (Sigma-Aldrich) in water was slowly added for 5-7 min, then removed. For washing, the wells were filled completely with PBS and incubated for at least 3 hours. Wells were either used immediately or stored at 4°C.

### Conditioned medium (CM) preparation

MSC or SB623 cells were plated at 3x10⁴cells/cm² and grown in αMEM supplemented with 10% FBS and penicillin/streptomycin for 3-4 days until confluence. Then the medium was replaced with Neurobasal medium (Invitrogen), and the cultures were incubated for 1-2 hours. This medium was discarded and replaced with fresh Neurobasal medium, using half of the volume typically used for cell growth. The cells were incubated for 24 hours, after which the medium was collected, and particulate matter was removed by centrifugation. The medium was dispensed in aliquots and stored at -70C°. MSC-CM preparations were supplemented with 2% B27 and 0.5 mM GlutaMAX before use.

### Preparation of rat embryonic brain cortical cells

Rat embryonic (E18) brain cortex pairs were purchased from BrainBits (Springfield, IL); and a cell suspension was prepared as described [29]. Briefly, cortices were incubated with 0.25% Trypsin/ EDTA at 37°C for 5-7 min, and trypsin was removed. The tissue was washed with αMEM containing 10% FBS, then with PBS. DNase (MP Biomedicals, Solon, OH) at 0.25 mg/ml was then added, and the contents of the tube were mixed by vortexing for 30 sec. The resulting cell suspension was triturated, diluted with PBS, pelleted and then resuspended in neural growth medium (described above).

### Co-culture experiments

Plates, coated as described above, were pre-warmed with a portion of neural growth medium, and then varying numbers of mesenchymal cells were added. Subsequently, neural cells were added at a density of 1.5x10⁴ cells/cm² to all but control wells, and cultures were incubated for the indicated time periods. For each time point, a separate plate was used that included quadruplicate samples. For quantitation, cells were plated in 96-well plates and MSC or SB623 cells were added at decreasing densities, starting at 1.5x10³ cells/cm² (*i.e.,* 500 cells per well) For immunostaining, cultures were plated on ECM-coated cover slips in 12-well plates. MSC or SB623 cells were added at a constant cell density of 1.5x10³ cells/cm² unless indicated otherwise.

In a subset of experiments, in which the effects of cells (MSC or SB623) were compared to the effects of their conditioned medium, a cryopreserved aliquot of cells was used to generate the conditioned medium prior to an experiment, and an aliquot of cells from the same donor was thawed on the day of experiment to generate a corresponding cell suspension. Cells were applied at decreasing concentrations as described above and conditioned medium was used at decreasing concentrations starting from 50% of total medium. For quantitation of gene expression, all culture conditions were tested on the same PCR plate using the same standard curve for each neural marker.

Medium was not changed during co-culture experiments (which lasted for 7- 8 days in 96-well format and for up to 14 days with the cells on cover slips). No signs of culture decline were noticed and the viability of neural cells was above 95% when assessed on the last day of culturing using Trypan Blue exclusion.

In another set of experiments, when cells were co-cultured under non-adherent conditions, Ultra-Low Adhesion Costar 12- or 24-well plates were used. Mesenchymal and neural cells were mixed in the indicated quantities and plated in neural growth medium. As a control, neural cells were grown alone or in the presence of 20 ng/ml or 50 ng/ml each of EGF and FGF2 purchased from either R&D Systems (Minneapolis, MN) or Peprotech (Rocky Hill, NJ). Medium was not changed over the course of the experiment (2 weeks).

### Immunocytochemistry

Cultures that were grown on glass cover slips were fixed with 4% paraformaldehyde (Electron Microscopy Science, Hatfield, PA) for 20 min, washed once with PBS and incubated for 30 min in blocking solution containing 10% normal donkey serum (Jackson Immunoresearch, West Grove, PA), 1% bovine serum albumin (Sigma-Aldrich), 0.1% Triton X-100. Then a goat polyclonal antibody against rat Nestin (R&D Systems, Cat #AF2736) was added into the blocking solution at 1:1000 and incubated overnight at 4C°. Cover slips were washed with PBS and then either rabbit polyclonal anti-glial fibrillary acidic protein (GFAP) (Dako, Denmark) (1:2000), mouse monoclonal anti-microtubule-associated protein 2 (MAP2) (Sigma-Aldrich) (1:1000), or mouse monoclonal anti- 2', 3'-cyclic nucleotide 3'-phosphodiesterase (CNPase) (Millipore, Billerica, MA) (1:200) was added and the cover slips were incubated for 1 hour at room temperature. After washing, cover slips were incubated for 1 hour with secondary antibodies: DyLight 488-conjugated AffiniPure donkey anti-goat F(ab')₂ fragments of IgG (1:1000) in combination with either DyLight 549 488-conjugated AffiniPure anti-rabbit F(ab')₂ fragments of IgG (1:2000) or Cy3-conjugated AffiniPure donkey anti-mouse IgG (1:1000), all from Jackson Immunoresearch, and all selected for use in multiple labeling by the manufacturer. After washing with PBS and water, the slips were mounted with ProLong Gold antifade reagent containing 4',6-diamidino-2-phenylindole (DAPI) (Invitrogen).

In some experiments, prior to fixation, cells were incubated for 7-8 hours with 10 uM 5-bromo-2'-deoxyuridine (BRDU, from Sigma-Aldrich) with or without mitomycin at a concentration of 50 ug/ml. Cultures then were fixed with 2% PFA, permeabilized with 0.5% Triton and treated with deoxyribonuclease (MP Biomedicals, Solon, OH) in a buffer containing 0.15 M NaCl and 4.2 mM MgCl₂ for 1 h at 37°C. The cultures were then post-fixed with cold methanol (Fisher Scientific, Fair Lawn, NJ) for 10 min. After blocking as described above, the cultures were incubated with anti-BRDU monoclonal antibody (BD Pharmingen), then with the anti-mouse secondary antibody described above, then with Alexa Fluor-conjugated TUJ1, a Neuronal Class III β-Tubulin-specific antibody (Covance, Princeton, NJ).

Fluorescence microscopy was conducted using a Nikon Eclipse50i (Nikon Instruments, Melville, NY) and a Nikon Digital Camera DXM1200C.

Under the conditions described herein, none of antibodies reacted with the mesenchymal cells.

### Gene expression quantification

Growth and culture of cells, for quantitation of expression of mRNAs encoding various neural markers, was conducted in 96-well plates. After culturing for the indicated time, the culture medium was carefully and completely aspirated using a Nunc ImmunoWasher equipped with 10 ul pipette tips; and cells were lysed with 20 ul/well of lysis buffer, either Cell-to-Signal™ (Applied Biosystems/Ambion, Austin, TX) or SideStep™ (Agilent Technologies, Santa Clara, CA) for 3 min. Then the lysates were carefully pipetted up and down and samples (in quadruplicate) were combined pair-wise (thus making biological duplicates), transferred to a storage plate and frozen at -70C°.

For gene expression testing, samples were thawed and aliquots were diluted 1:10 with PCR-grade water. A sample with a high expected expression level was also used to prepare serial dilutions in 10% lysis buffer to serve as a series of standards for the quantification. The diluted samples were used as templates in one-step qRT-PCR reactions, in combination with QuantiTect® Probe RT-PCR Master Mix from Qiagen (Valencia, CA) and TaqMan® gene expression assays purchased from Applied Biosystems (Foster City, CA). The absence of cross-reaction between corresponding mRNAs from rat and human cells was established in experiments involving rat neural cells, as well as human mesenchymal and neural cells. The following pre-optimized assays, all designed across exon-exon boundaries, were used: rat-specific - nestin (Rn00564394_m1), MAP2 (Rn00565046_m1), GFAP (Rn00566603_m1), CNPase (Rn01399463_m1), Doublecortin (Dcx)(Rn00584505_m1), glyceraldehyde 3-phosphate dehydrogenase (rGAP)(Rn-1462661_gl); and human-specific - glyceraldehyde 3-phosphate dehydrogenase (huGAP) (4333764F), bone morphogenetic protein 4 (BMP4) Hs00370078_m1, and fibroblast growth factor 2 Hs00266645_m1. Numbers in parentheses refer to the manufacturer's (Applied Biosystems) assay ID numbers. For amplification reactions, a LightCycler 480 (Roche, Mannheim, Germany) was programmed according to the Master Mix manufacturer's protocol, with 40-60 amplification cycles. Analysis was done using a Second Derivative Maximum method.

### Assessment of neuritogenesis

Neural cells were plated at 1.5x10⁴ cells/cm² alone or together with either MSC or SB623 cells at 1.5x10³ cells/cm² on ECM-covered glass cover slips in neural growth medium containing 10-fold less B27 than normally used (0.2%), and allowed to grow for 18-24 hrs. Then the cultures were fixed and stained for MAP2 expression and mounted with DAPI-containing medium, as described above. Approximately 12-15 fields were photographed using the same exposure time, and a total of 20 neurons per condition were analyzed. The length of the longest neurite was measured for each cell and the number of neurites per cell was counted.

### Example 1: Effect of mesenchymal cells on proliferation and neuronal differentiation

The composition of ECM-based neural cultures grown with or without mesenchymal cells was first analyzed using immunocytochemistry for Nestin, a marker of neural stem cells/early progenitors, and MAP2, a neuronal marker. At various times cultures were fixed and incubated with rat-specific anti-Nestin antibody and anti- MAP2 antibody. All cultures were also counterstained with the nucleus-specific dye DAPI. Fixed and stained cultures were then examined by fluorescence microscopy. On day 1, single positive Nes⁺ and MAP2⁺ cells were present in all cultures tested, along with some MAP2⁺Nes⁺ double-positive cells, in which MAP2 staining and Nestin staining were co-localized. There was also a small fraction of double-negative cells. By day 3, no double-positive cells were detected, while single-positive cells extended their processes. On day 5, MAP2⁺ neurons continued to extend neurites, while Nes⁺ cells were significantly increased in numbers. At this time point, Nes⁺ cells formed colonies. A greater number of colonies, and larger colony size, were observed in the presence of mesenchymal cells. Double-positive cells were not detected.

By day 9, a large number of MAP2⁺Nes⁺ double-positive cells were observed, as well as MAP2⁺Nes⁻ neurons and MAP2⁻Nes⁺ cells. The double-positive cells were found in greater numbers in co-cultures with SB623, compared to co-cultures with MSC, while cultures without mesenchymal cells had the smallest number of MAP2⁺Nes⁺ cells. These double-positive cells had nuclei of a characteristic bilobular shape, thin MAP2-positive processes, and a strong Nestin reactivity localized to a perinuclear area - most frequently, to a cleft between two nuclear lobes situated adjacent to the most prominent outgrowth. This morphology resembled that of the MAP2⁺Nes⁺ double-positive cells present on the first day of culturing.

When neural cells and mesenchymal cells were co-cultured on PDL, approximately the same frequency of double-positive and single-positive cells were observed on Day 1 of culture as were observed when cells were co-cultured on ECM. At later time points, no developed single-positive Nes⁺ cells were detected (very rare Nes⁺ cells were round, with dense nuclei). By Day 5 of culture, only a small number of double-positive cell colonies, each consisting of very few cells, were observed. These results indicate that, on PDL, most or all Nes⁺ cells were committed to neuronal differentiation. At day 9, double-positive colonies were more prominent in the presence of mesenchymal cells than in their absence.

The status of mesenchymal cells in co-cultures was also examined using phase contrast microscopy and staining for α-smooth muscle actin, a mesenchymal marker. On PDL, mesenchymal cells were barely spread and usually disappeared around day 5-7. On ECM, mesenchymal cells were easily detected throughout the duration of co-culturing; they appeared well-spread, moving, and appeared to be slowly proliferating.

Neuritogenesis was very active on both ECM and PDL in co-cultures, but was further enhanced in the presence of mesenchymal cells during first 18-24 hrs in culture. To increase this differential response, cultures were plated in neural growth medium with a low concentration of B27 supplement. Under these conditions, longer neurites were observed in the presence of MSC or SB623 cells after 24 hours of co-culturing, than in cultures of neural cells alone (Table 1). However, no significant difference in numbers of neurites was noticed (Table 1).

**Table 1: Length and Numbers of Neurites on Day 1**

| | ECM (n=19) | | ECM + MSC (n=23) | | ECM + SB623 cells (n=22) | |
|---|---|---|---|---|---|---|
| | length | number | length | number | length | number |
| Median | 15 | 2 | 18 | 3 | 25 | 3 |
| Average | 15.3+/-6.3 | 2.6 | 22+/-13.9 | 2.6 | 31.6+/- 19.7 | 3.1 |
| Maximum | 25 | | 70 | | 85 | |

| | | | | | | |
|---|---|---|---|---|---|---|
| Neurite length is expressed in µm | | | | | | |

### Example 2: Effect of mesenchymal cells on astrogenesis

Astrocyte development was assayed by immunohistochemical analysis for expression of glial fibrillary acidic protein (GFAP). Double-staining of ECM-based cultures for glial fibrillary acidic protein (GFAP, an astrocyte marker) and Nestin revealed the absence of GFAP reactivity before day 3 in all cultures. Around day 5, GFAP-expressing cells began to be observed in co-cultures within Nes⁺ colonies as single- or double-positive cells. GFAP-expressing cells were not observed in cultures not containing mesenchymal cells. Different colonies had variable proportions of GFAP⁺Nes⁺ cells and the more fully differentiated GFAP⁺Nes⁻ cells. No GFAP⁺ cells were detected at this time in cultures lacking mesenchymal cells. On day 9, all three phenotypes (GFAP⁺Nes⁺, GFAP⁻Nes⁺, and GFAP⁺Nes⁻) were present in all cultures, with GFAP⁺Nes⁻ cells predominating.

With respect to its intracellular localization, GFAP immunoreactivity appeared initially (at day 5) as intercalating filaments beside or within Nestin filaments in some Nestin-positive filamentous cells. Later, Nestin was practically displaced by GFAP in certain cells, as evidenced by a patchy distribution of Nestin staining, while other cells continued to express Nestin only. In co-cultures, GFAP⁺Nes⁺ cells had generally the same morphology as GFAP⁺Nes⁻ cells, while among GFAP⁻Nes⁺ cells, morphology varied. One type of cell had very long processes, frequently exceeding 200 µm. Other types were small GFAP⁻Nes⁺ cells, with Nestin reactivity localized eccentrically with respect to the nucleus, either as a "lace" from one side of nucleus, or concentrated in a cleft of a bilobular nucleus and extended into a process positioned against the cleft. The latter morphology was similar to that of Nes⁺MAP2⁺ cells described in the previous example.

No GFAP⁺ cells were detected when co-cultures were conducted on PDL.

### Example 3: Effect of mesenchymal cells on oligodendrogenesis.

Oligodendrogenesis was assessed by immunohistochemical analysis of expression of the early oligodendrocyte marker 2', 3'-cyclic nucleotide 3' phosphodiesterase (CNPase). CNPase⁺ cells could not be detected before day 9 of co-culture. On day 12, in cultures grown on ECM in the absence of mesenchymal cells, CNPase reactivity could be detected only in a very few, usually dividing, cells. At the same time point, in co-cultures with mesenchymal cells, CNPase⁺ cells appeared in clusters, with CNPase expression localized to the perinuclear area. In co-cultures with SB623 cells, CNPase staining was both more intense and more extensive, extending throughout the cytoplasm. Expression of CNPase did not co-localize with Nestin expression.

No CNPase⁺ cells were detected when co-cultures were conducted on PDL.

### Example 4: Neural cell proliferation in co-cultures

Proliferation of neural cells was assayed in cultures containing 1.5x10⁴ neural cellscells/cm², with or without MSC at 1.5x10³ cells/cm², using two methods. In the first method, DAPI-stained nuclei were counted on slides prepared for immunochemistry analysis as described above. Five microscopic fields at 200x-magnification were counted and averaged per condition, from 2 experiments. Extremely condensed or fragmented nuclei were considered to be indicative of dead cells. MSC nuclei were excluded from counting based on their distinctive size.

In the second method, neural cell proliferation was measured in microplate format co-cultures using a quantitative PCR assay for rat noggin, a single-exon gene (Rn01467399_s) (Applied Biosystems). The analysis was conducted as described for qRT-PCR, with the exception that the reverse transcription step of the qRT-PCR protocol was omitted. Minimal amplification of human noggin sequences from the MSC was detected (Figure 1B).

The results are shown in **Figure 1****.** Both methods indicated that, in the presence of MSC, the number of rat neural cells tripled to quadrupled over the course of 7 days of co-culture with MSC, while in the absence of MSC, neural cell number barely doubled. As assessed by morphology of DAPI-stained neural nuclei, 10-20% of cells were dead at any given time (Figure 1A).

Proliferation of neural precursor cells was also assayed in co-cultures of rat neural cells with MSC on ECM. On day 7 of co-culture, cultures were treated with BRDU for 7 hours following by fixing and immunostaining with antibody to BRDU and with the neuron-specific TUJ1 antibody. Irrespective of whether neural cells were cultured alone or co-cultured with MSC, at this time point the cultures contained small cells, with barely developed processes, exhibiting reactivity with both anti-TUJ1 and anti-BRDU antibodies, indicative of a proliferating neural precursor cell. Quantitation of these neural precursor cells, using a PCR assay for the rat *noggin* gene, showed that their numbers were increased when the neural cells were co-cultured with MSC (Figure 1B).

### Example 5: Time course

In this example, the time course of expression of various neuronal (doublecortin, MAP2), neural precursor (nestin) and glial markers (GFAP and CNPase), in neural cells cultured on ECM, was analyzed in the presence and absence of 200 MSC/well. Samples were collected at various time points and frozen. All samples were subsequently thawed and assayed in parallel by qRT-PCR. Primers, probes and amplification conditions were as described above. The results are shown in **Figure 2****.** Levels of the neural markers doublecortin (DCX) and MAP2 are initially high, and increase with time in culture and co-culture. At intermediate culture times, co-culture seems to have little effect on DCX and MAP2 RNA levels; while, at later times, the activating effect of co-culture becomes significant. Expression of nestin, a neural precursor cell marker, was almost undetectable at the initiation of co-culture but increased steadily over seven days of culture, and was enhanced by the presence of MSC. Expression of GFAP mRNA, an astrocyte marker, was not detected in the absence of co-culture with MSC; while in co-cultures it was first detected on Day 4, with a large increase in expression between Days 6 and 7. Expression of CNPase mRNA, an oligodendrocyte marker, was first detected on Day 4 in co-cultures with MSC, and also exhibited a sharp increase between Days 6 and 7.

Based on these results, the optimal time for detection of Nestin and CNPase mRNA expression was determined to be Day 5 of co-culture; and the optimal time for detection of DCX, MAP2 and GFAP mRNA expression was determined to be Day7 of co-culture.

### Example 6: Dose Response

For quantitative assays, rat cortex cells (5000 cells/well) were cultured alone or co-cultured with decreasing numbers of MSC, from 500 to 32 cells/well. As a control, MSC were also cultured alone at 500 cells/well. qRT-PCR Taqman assays for rat Nestin, MAP2, GFAP, and CNPase mRNAs were used to quantify gene expression. A human-specific glyceraldehyde 3-phosphate dehydrogenase (huGAP) qRT-PCR Taqman assay was used to estimate MSC numbers. **Figures 3** **and** **4** show that total levels of rNes, rMAP2, rCNPase, or rGFAP gene expression in co-culture samples were directly dependent on the number of MSC present, MSC number being quantified by assay for human-specific GAP (huGAP) mRNA. These effects were not caused by the amplification of human sequences since MSC alone gave no signal using rat-specific PCR probes and conditions.

To observe a MSC-dependent dose response using Nestin, MAP2, or CNPase as markers, the timing of sampling was important. For example: the optimal timing for testing rat Nestin gene expression was between day 4 and 6, since on day 7 the expression reached saturation.

Rat CNPase mRNA expression was first detected around day 5, long before the protein could be detected. At Day 5, CNPase mRNA levels were directly proportional to MSC concentration in co-cultures. After day 5, CNPase gene expression levels continued to increase; but by Day 7, the MSC-dose-dependence curve became biphasic (**Figure 5**). At these later times, higher doses of MSC progressively inhibited CNPase gene expression over the course of the observation, with lower doses remaining inductive. This result was confirmed at the protein level: neural cells co-cultured with 1000 cells/cm² of MSC or SB623 had significantly less CNPase staining, compared to neural cells co-cultured with 100 cells/cm² of MSC or SB623 cells.

Expression of GFAP mRNA showed a strong and consistent dose response to mesenchymal cell concentration, as soon as it could be detected (day 4 or 5) and did not demonstrate saturation for the remainder of the culture period (days 7-9).

### Example 7: Quantitation of effects mediated by mesenchymal cell conditioned medium, mesenchymal cell ECM, and live mesenchymal cells

In this example, the effects of live MSC on neural cell differentiation were compared to the effects of their conditioned medium (CM); and the effects of using ECM as a substrate were compared to the use of poly-D-lysine.

Quantitative neural differentiation assays (qRT-PCR) were used to determine which components of mesenchymal stem cell cultures had the greatest neuropoietic effects. For this purpose, the response of neural cells to MSC was compared to that to MSC-CM, and cells were co-cultured either on ECM- or PDL-coated plates. Decreasing concentrations of MSC and MSC-CM were used to ensure that effects were analyzed below saturation. The results are summarized in Table 2. To simplify the presentation, the table includes only the data from experiments that included the highest concentration of mesenchymal cells (500 cells/well) or MSC-CM (50%). Lower concentrations of these additives stimulated lower marker expression levels, confirming that the responses were neither saturated nor down-regulated. The results were expressed relative to the levels in cultures grown on ECM without additives on the indicated day.

Similar levels of rMAP2 expression on day 1 were observed under all test conditions indicating that initial neuron attachment and development was similar. Later, on day 5, the presence of either live MSC or MSC-CM increased the expression of this marker 2-3-fold on either substrate. Nestin gene expression was 2-3 orders of magnitude stronger on ECM than on PDL. On day 5, Nestin gene expression was substantially increased in the presence of both MSC-CM and live MSC on both ECM and PDL. CNPase gene expression on day 5 was induced by live MSC and induced even more strongly by MSC-CM on ECM-based cultures, while on PDL-based cultures, it was below detection limits. CNPase gene induction could be detected on PDL on day 7, with MSC-CM being a more effective stimulus than live MSC. GFAP gene expression was induced on ECM most dramatically by live MSC and, to a lesser extent, by MSC-CM. On PDL, GFAP expression was below quantification limits throughout the study.

Human GAP expression was also tested on day 1 and on day 7 of this study. On day 1, human GAP gene expression in co-cultures conducted on PDL-coated plates was only slightly lower than in cells cultured on ECM, while on day 7 it was below quantification limits. Microscopic examination revealed that, after 7 days on PDL, only a few mesenchymal cells survived, and they were barely spread, while on ECM they exhibited their typical morphology and had slightly increased in number. The results are summarized in Table 3.

**Table 2: Relative Expression Levels of Neural Markers on ECM and PDL**

| | ECM | | | PDL | | |
|---|---|---|---|---|---|---|
| | No add | MSC cells* | MSC-CM** | No add | MSC cells* | MSC-CM** |
| MAP2 d 1 | 1 (11%) | 1 (6%) | 1.2 (18%) | 0.4 (50%) | 0.5 (2%) | 0.9 (25%) |
| MAP2 d5 | 1 (3%) | 2.2 (9%) | 3.5 (14%) | 1 (1%) | 2.8 (7%) | 3.5 (7%) |
| Nestin d5 | 1 (6%) | 3.8 (3%) | 8 (8%) | 0 | 0.001 (10%) | 0.031 (60%) |
| Nestin d8 | 1 (26%) | 3.4 (3%) | 2.9 (21 %) | 0.012 (100%) | 0.086 (23%) | 0.024 (45%) |
| GFAP d7 | 1 (91%) | 7727 (1%) | 1636 (11%) | 0 | 0 | 1.8 (100%) |
| CNPase d5 | 1 (100%) | 20 (5%) | 75 (7%) | 0 | 0 | 0 |
| CNPase d7 | *S*/*I* | *S*/*I* | *S*/*I* | 0.2 (25%) ^{#} | 7.0 (21%) ^{#} | 7.5 (33%) ^{#} |

| | | | | | | |
|---|---|---|---|---|---|---|
| Values for each marker are shown relative to values from "No add, ECM" for each day. Coefficients of variations are indicated in parentheses. * MSC cells plated at 500 cells/well ** MSC-CM at 50% ^{#} Relative to the "No add, ECM" sample from day 5 *S*/*I* Signal is saturated or inhibited at these time points | | | | | | |

**Table 3: Summary of observations on effects of human MSC, MSC-CM, and MSC-derived ECM on neuropoiesis in rat E18 cortical cell cultures**

| | PDL | ECM |
|---|---|---|
| No add | N, Nn, S* | N, S+, Nn+, A+, O+ |
| MSC, cells | N, S*, Nn+, O*, A | N, S+++, Nn+++, A+++ O+++ |
| MSC, CM | N, S*, Nn+, O* | N, S+++, Nn+++, A++, O+++ |

| | | |
|---|---|---|
| Growth of each cell population estimated and expressed by "+". Abbreviations: A, astrocytes; O, oligodendrocytes; N, existing neurons, likely not proliferating; Nn, newborn neurons (Nes+), proliferating; S, neural stem/early progenitors. * Transcription of the marker can be detected, while the protein is undetectable, or only very few positive cells are detected during 9 days of culturing. | | |

### Example 8: Effects of MSC in non-adherent cultures

In the experiments described above, the ECM coating of plates was produced by confluent layers of mesenchymal cells whose concentration was approximately 40-fold greater than the highest concentration of mesenchymal cells used in the co-cultures. To clarify whether the strong stimulation of neural stem/early progenitor cell proliferation and differentiation in co-cultures was caused by the presence of mesenchymal ECM in co-cultures, cortical cells were mixed with decreasing numbers of MSC or SB623 cells and co- culture was conducted under non-adherent conditions, in plates coated with poly-D-lysine (PDL). As controls, cortical cells were plated alone, with or without FGF2 and EGF; and mesenchymal cells were plated alone at the highest concentration used in co-culture.

For poly-D-lysine (PDL) coating, plates were coated with PDL (Sigma-Aldrich) at 10 µg/ml in water for 1 hour at room temperature. Then the PDL solution was aspirated, wells were allowed to dry, and then washed once with PBS. Before cells were plated in these coated wells, the PBS was replaced with a portion of the neural growth medium, and the plates were warmed in an incubator during cell suspensions preparation.

Cells were co-cultured for 14 days. During this time, cell aggregates were formed in all wells. In cultures of mesenchymal cells alone, these aggregates were small and no visible increase in their size was observed during the culture period; indeed, many of them died. Under all other conditions, aggregates grew significantly, forming typical neurospheres. At the end of the incubation, the total contents of all wells was collected, pelleted, and lysed in equal volumes of lysis buffer; then tested by qRT-PCR for the expression of rat neural markers. **Figure 6** provides representative results, showing that the presence of MSC strongly stimulated rNes, rMAP2, rGFAP, and rCNPase expression in a dose-dependent fashion under non-adherent conditions in the absence of an ECM. The expression of rat doublecortin (rDCX, a marker of proliferating neurons) was also stimulated by MSC on the PDL-coated substrate. MSC also induced expression of rGAP in a dose-dependent fashion, indicating that co-culture with MSC stimulated an increase in the overall numbers of viable rat neural cells.

When neurospheres observed in co-cultures containing the highest dose of MSC were compared to neurospheres grown in the presence of FGF2 and EGF, the former had one-third the level of Nestin gene expression, but 2.5-fold higher Dcx expression and 55-fold higher GFAP expression, as well as higher levels of MAP2, CNPase, and rGAP expression (1.5, 3.5, and 3 times, respectively). Human GAP (huGAP) mRNA was detected in MSC cultured alone under non-adherent conditions, but its levels were dramatically reduced when the same number of cells were co-cultured with neural cells (17.2 and 3.1, respectively). Lower doses of MSC exhibited huGAP expression levels that were below quantification limits. This indicated that the neural cell environment, in combination with non-adherent conditions, was unfavorable for growth of mesenchymal cells, and they did not survive at lower plating doses. Nevertheless, their ability to stimulate neural development persisted.

In cortical cells cultured in the absence of bFGF and EGF, gene expression of all neural markers was low, as was expression of rat GAP. In co-cultures with MSC, expression of all markers was increased in a dose-dependent fashion, despite the fact that the majority of MSC died in co-cultures, as well as when cultured alone (as indicated by huGAP levels).

Similar results were obtained using SB623 cells instead of MSC. These results show that the stimulatory effects of mesenchymal cells observed in co-cultures on ECM are not due to the ECM itself.

### Example 9: Effect of attachment conditions

The effects of attachment conditions on the differentiation of neural cells in co-culture were assessed. To this end, neural cells were co-cultured with MSC on SB623 cell derived ECM-coated plates, on ornithine/fibronectin-coated plates, and on Ultra Low Attachment (ULA) plates (Corning, Lowell, MA). Ornithine/fibronectin-coated plates supported attachment of MSC. On ULA plates, neural cells were cultured either with a five-fold higher concentration of MSC (compared to co-culture on ECM or ornithine/fibronectin) or with 20 ng/ml each of fibroblast growth factor-2 (FGF2) and epidermal growth factor (EGF).

Ornithine/fibronectin coating (Orn/FN) was prepared by incubating wells with 15 ug/ml poly-L-ornithine (Sigma-Aldrich) in PBS, overnight at 37°C, then washing the wells 3 times, followed by incubation overnight with PBS at 37°C. After this, wells were incubated with 1 ug/ml bovine fibronectin (Sigma-Aldrich) in PBS, for 3-30 hours and washed once before plating cells.

A mixed suspension of rat cortical cells and human MSC (neural cells/MSC ratio 10:1, 1.5x10⁴/cm²) was plated on SB623 cell ECM-coated plates and on ornithine/fibronectin-coated plates. Neural cells mixed with either a five-fold higher concentration of MSC or with FGF2 and EGF (as described above) were plated on ULA plates. Marker expression was assayed by qRT-PCR at either 5 or 7 days of co-culture.

The results are shown in **Figure 7****.** Neural cells co-cultured with MSC on ECM expressed significantly higher levels of GFAP, compared to all other conditions. Nestin expression on ECM-coated plates was significantly higher than on plates coated with Orn/FN, and was similar to that observed in cells cultured on ULA plates, either stimulated with recombinant cytokines, or co-cultured with high concentrations of MSC. In 7 days, in co-cultures grown on ECM, rat Nestin and GFAP expression levels were significantly higher than on Orn/FN, while rat DCX, CNP, and human GAP expression levels were similar. Co-cultures grown on ECM exhibited significantly higher expression of GFAP and DCX than did non-adherent co-cultures.

Non-attached growth (on ULA plates) had a detrimental effect on growth and survival of MSC, as evidenced by the fact that huGAP levels were very low despite a 5-fold greater MSC concentration on ULA plates compared to ECM and orn/FN-coated plates. Under non-adherent conditions, FGF2/EGF and MSC supported similar levels of Nes and CNPase expression.

In summary, co-cultures of neural cells and MSC grown on ECM-coated plates contained the most diverse neural cell population, in contrast to co-cultures on Orn/FN or in ULA wells. In particular, co-culture on ECM supported levels of nestin expression that were comparable to those in FGF2/EGF-driven spheroids, and also supported the highest levels of GFAP expression under any of the conditions tested.

### Example 10: Role of heparan sulfate proteoglycans

In light of the positive effects of ECM on the abundance of nestin-expressing cells, as described in the preceding example, the effect of the heparan sulfate proteoglycan components of the ECM on nestin expression were investigated.

For these experiments, plates were coated with SB623 ECM as described *supra,* then ECM was treated with a solution of Heparinase 1 (Sigma-Aldrich) in 10 mM HEPES, pH 7.4, 100 mM NaCl, and 4 mM CaCl₂ overnight at room temperature and washed once. Heparinase concentrations are given in the legend to Figure 8.

Rat cortical cells were cultured on the plates containing heparinase-treated ECM. After five days of culture, nestin expression was assayed by qRT-PCR. The results, shown in **Figure 8****,** indicate that treatment of ECM with heparinase results in a heparinase-dose-dependent reduction in nestin expression. From these results, it can be concluded that heparan sulfates contribute to the growth of nestin-expressing neural cells.

### Example 11: Expression of Growth Factors and Cytokines by MSC

Quantitative RT-PCR was used to measure the expression, by MSC, of mRNA encoding certain growth factors and cytokines, as shown in Table 4 below.

**Table 4**

| | **Crossing point*** | **Standard Deviation** |
|---|---|---|
| **BMP-2** | 35.8 | 0.3 |
| **BMP-4** | 31.3 | 1.0 |
| **BMP-6** | 33.2 | 0.6 |
| **FGF-1** | 31.1 | 0.2 |
| **FGF-2** | 27.7 | 0.6 |
| **FGF-2AS** | 31.5 | 0.5 |
| **FGFR-2** | 27.0 | 0.3 |
| **EGF** | 29.6 | 0.5 |
| **HBEGF** | 29.3 | 0.4 |
| **IGFBP5** | 26.1 | 0.8 |
| **GAP (control)** | 21.8 | 0.4 |

| | | |
|---|---|---|
| * - The amplification cycle at which signal is first detected | | |

### Example 12: Assay for neurogenic and/or gliogenic effects of cytokines, growth factors and other proteins

A number of growth factors and cytokines produced by MSC were tested for their ability to stimulate neurogenesis and gliogenesis, by adding the recombinant factor, either by itself or with 5% MSC conditioned medium (MSC-CM), to neural cells cultured on ECM.

ECM was produced by growing SB623 cells in culture, then washing the cells from the culture vessel. Primary embryonic rat cortical cells (Brain Bits, Springfield, IL) were cultured on the ECM in the presence of 5% MSC conditioned medium and a particular recombinant cytokine or growth factor, as shown in Table 5 below, for 5 or 7 days. The cells were then assayed for the expression of various rat neuronal and glial markers by species-specific quantitative RT-PCR. A summary of these results is shown in Table 5.

**Table 5**

| | **Nestin** | **MAP2** | **GFAP** | **CNPase** |
|---|---|---|---|---|
| FGF-1 | + | + | + | + |
| FGF-2 | + | + | - | + |
| BMP-2 | - | | + | |
| BMP-4 | - | | + | |
| BMP-6 | - | | + | |
| EGF | + | + | - | + |
| HB-EGF | + | | | + |
| HGF | + | | | + |
| IL6 | | +/- | +/- | +/- |
| IL8 | +/- | | +/- | +/- |
| ILlb | +/- | +/- | +/- | + |

| | | | | |
|---|---|---|---|---|
| + = increase +/- = weak induction - = decrease | | | | |

Quantitative results showing the effects of three factors (EGF, BMP6 and HB-EGF) on expression of markers for neuronal precursors (Nestin), nascent neurons (DCX), oligodendrocytes (CNPase) and astrocytes (GFAP) are shown in **Figure 9****.**

### Example 13: Role of FGF2 in upregulation of nestin expression

Fibroblast growth factor-2 (FGF2) was secreted by MSC (Table 4, *supra*) and addition of FGF2 to cortical cells stimulated expression of nestin, MAP2 and CNPase (Table 5, *supra*). Two additional experiments were conducted to confirm the role of FGF2 in stimulating nestin expression. In the first, a blocking antibody to FGF2 was added to co-cultures of neural cells and MSC. In the second, MSC conditioned medium was depleted of FGF2 and added to cultures of neural cells.

For the first experiment, two antibodies were used: bFM1 (a FGF2 neutralizing antibody that recognizes both rat and human FGF2) and bFM2 (a FGF2-specific non-neutralizing antibody), both obtained from Millipore, Billerica, MA. Rat cortical cells were cultured at a concentration of 5,000 cells/well in the presence or absence of MSC at a concentration of 200 cells/well. The antibodies were added to co-cultures of cortical cells and MSC at a concentration of 0.2 ug/ml.

The results of this analysis are shown in **Figure 10****.** Co-culture of neural cells with MSC enhances nestin expression by the neural cells, as expected. However, when co-culture was conducted in the presence of the anti-FGF2 neutralizing antibody, nestin expression was reduced to a level below background. The presence of the non-neutralizing anti-FGF2 antibody in the co-culture had little to no effect on MSC-dependent upregulation of nestin expression in neural cells in the co-culture.

For the second experiment, FGF2-depleted MSC-CM, and control MSC-CM, were prepared as follows. MSC-CM was incubated with the anti-FGF2 neutralizing antibody bFM1, or with control mouse IgG1, at 5 ug/ml overnight at 4°C on a rotisserie shaker, followed by the addition of protein A/G-plus Agarose (Santa Cruz Biotechnology, Santa Cruz, CA) and incubation for 1 hour. After removal of the beads by centrifugation, the supernatant was collected and sterile-filtered.

Neural cells were cultured on ECM-coated plates with no further additions, or with addition of MSC-CM, FGF2-depleted MSC-CM, or control immunoprecipitated MSC-CM, for 5 days, and nestin mRNA expression was measured by qRT-PCR. The results are shown in **Figure 11****.** Addition of MSC-CM to neural cells resulted in increased nestin expression, as expected. However, FGF2-depleted MSC-CM had little, if any, stimulatory effect on nestin expression. MSC-CM that had been subjected to the same immunoprecipitation procedure using a non-FGF2-specific antibody stimulated nestin expression to the same extent as untreated MSC-CM.

These results indicate that MSC-derived FGF2 is a primary factor responsible for nestin induction in neural cells, and also indicate that basal nestin levels in cortical ECM-based cultures were dependent on FGF2, either of rat or human origin.

### Example 14: Role of MSC-derived factors in astrocyte development

The effect of mesenchymal stem cells was compared with the effect of conditioned medium from mesenchymal stem cells on the expression of nestin and GFAP in neural cell cultures on ECM-coated plates. As shown in **Figure 12****,** similar levels of nestin mRNA were induced by 200 MSC per well and by 10% MSC conditioned medium. However, induction of GFAP expression by conditioned medium was lower than that induced by cells themselves. This result indicated that a component responsible for astrocyte development was less abundant (and/or less active) in MSC conditioned medium that in the MSC themselves.

Bone morphogenetic protein -4 (BMP4), a factor secreted by MSC (Table 4, *supra*), stimulated expression of GFAP when added to cultures of neural cells (Table 5, *supra*). To confirm the role of BMP4 in astrogenesis, co-culture of rat neural cells and MSC was conducted in the presence of a BMP agonist (noggin) and in the presence of an anti-BMP4 antibody. Recombinant human Noggin protein, obtained from R&D Systems (Minneapolis, MN), was included in the co-cultures at a final concentration of 30 ng/ml. Polyclonal goat anti-BMP4 and normal goat IgG control were used in co-cultures at 2 ug/ml. These reagents, as well as mouse IgG1 isotype control were obtained from R&D Systems (Minneapolis, MN).

**Figure 13** shows that the BMP antagonist *noggin* inhibited induction of GFAP expression in neural cells co-cultured with MSC on ECM-coated plates. Partial inhibition of GFAP induction by MSC was also observed when neural cells were co-cultured with MSC in the presence of an anti-BMP4 antibody (Figure 13).

Attempts were made to immunoprecipitate BMP4 from MSC conditioned medium by incubating MSC-CM with the anti-BMP4 antibody or control (goat IgG, or no antibody) at 5 ug/ml overnight at 4°C on a rotisserie shaker following by the addition of protein A/G-plus Agarose (Santa Cruz Biotechnology, Santa Cruz, CA) for 1 hour. After removing beads by centrifugation, the supernatant was collected and sterile-filtered. However, GFAP levels did not differ significantly in neural cells cultured in the presence of MSC-CM, compared to neural cells cultured in the presence of BMP4-depleted MSC-CM. Nonetheless, the anti-BMP4 antibody was capable of blocking GFAP induction driven by recombinant BMP4.

The lower astrogenic activity of MSC-CM compared to MSC; the partial decrease of GFAP levels in co-cultures treated with an anti-BMP4 neutralizing antibody; and the lack of effect of BMP4 immunodepletion on the astrogenic activity of MSC-CM, taken together, suggest either that the active BMP4 astrocyte-inducing activity resides within a cell-ECM compartment (rather than in the medium), or that it is produced by rat cells.

To test whether active BMP4 in co-cultures was produced by MSC or by the rat neural cells, production of BMP4 by the MSC was inhibited, prior to co-culturing, using siRNA. For siRNA transfection, freshly thawed MSC were plated at 0.4x10⁶ cells per 6-well plate in aMEM/10%FBS. Next day cells were transfected with either ON-TARGETplusSMARTpool human BMP4 siRNA or a control non-targeting pool at 25 nM, using DharmaFECT®1 (all reagents from Thermo Scientific Dharmacon®, Lafayette, CO) according to the manufacturer's instructions. Next day cells were trypsinized and lifted, and trypsin was inhibited by adding FBS. Cells then were washed twice in Neurobasal medium and counted (viability was usually greater than 95%).

The day after transfection, equal cell numbers of transfectants were plated with rat cortical cells and co-cultured for 5 days. On day 5, rat GFAP mRNA levels and human BMP4 levels were assayed. The results, shown in **Figure 14****,** indicate that rat GFAP mRNA levels were significantly reduced, and human BMP4 mRNA was virtually undetectable, in co-cultures containing MSC that had been transfected with BMP4-siRNA; while expression of the human GAP and FGF2 genes was not affected. Reduction of GFAP mRNA levels was not observed in cells transfected with control siRNA. These results strongly suggested that the BMP4 contributing to stimulation of astrogenesis in the co-cultures was MSC-derived.

### Conclusions and Observations

On ECM, the growth of Nes⁺ cells was significantly augmented in a dose-dependent fashion by live mesenchymal cells or their conditioned medium, as demonstrated using immunostaining and qRT-PCR, while on PDL the response to these factors was reduced (Figures 1 and 5 and Table 2). This suggests that the proliferation of Nes⁺ stem/early progenitor cells was stimulated by secreted mesenchymal cell-derived factors, and synergistically augmented by growth on mesenchymal cell ECM. The most likely mechanism for this synergy is the efficient accumulation, preservation, and presentation of mesenchymal cell-derived growth factors to neural cells by matrix proteoglycans. The conclusion that the proliferation of neural Nes⁺ cells can be stimulated by distantly acting MSC-derived soluble factors is in agreement with a recent report, which showed that mouse neurospheres co-cultured with mouse MSC, but separated from them by a semi-permeable membrane, had a high percentage of Ki-67-positive cells [38]. Indeed, MSC are known to secrete many growth factors that have been shown to participate in the maintenance of neural precursors in vivo [16, 30] and the secretion of some of them, including BMP4, FGF2, EGF, VEGF, and PDGF-AA has been confirmed in the MSC and SB623 cell batches used here [39].

Mesenchymal stromal cells in co-cultures promoted both neuritogenesis and *de novo* neuron formation from Nes⁺ cells (Figures 1 and 2; Tables 1 and 2). Both of these effects were observed using immunostaining for MAP2 proteins (MAP2 proteins are specific markers of neuronal cell bodies and neurites); and the combined effect was quantified using an expression assay for MAP2 mRNA. The enhanced neuritogenesis and increased numbers of Nes⁺MAP2⁺ cells were observed on both ECM and PDL in the presence of mesenchymal cells, indicating that the soluble mediators of both neuritogenesis and neuron formation do not appear to require ECM for their effects. Indeed, the addition of MSC-CM to either ECM- or PDL-based cultures elevated MAP2 gene expression as effectively as did the addition of live cells (Table 2). Neuritogenic effects of MSC were previously observed on neurons of different origin [16, 23, 40, 41].

Mesenchymal cells, including MSC and SB623, increased the formation of new neurons on ECM, as demonstrated by a massive appearance of double-positive Nes⁺MAP2⁺ cells around day 7 in the co-cultures (shown on Fig 1, day 9). In the absence of mesenchymal cells these double-positive cells appeared later and in smaller numbers. A rat MAP2 mRNA expression assay showed a significant increase of signal in a MSC-dose-dependent manner starting from day 5 (Figure 5) which was similar on ECM and on PDL (Table 2). Since this increase preceded the appearance of nascent neurons, the MSC dose-dependent increase in MAP2 gene expression likely reflected the proliferation of neuroblasts. Measurements of levels of mRNA for rat doublecortin (rDcx), a marker of proliferating neurons, yielded results similar to those for MAP2 expression (not shown), indicating that rDcx is likely expressed in both nascent and mature neurons, as is MAP2.

At the plating densities used here, GFAP expression was a hallmark of ECM-based cultures and was absent in PDL-based cultures. GFAP protein staining was closely associated with the staining for Nestin filaments in Nes⁺ filamentous or flat stellar-shaped cells around day 7 (Figures 3A and 3B). GFAP did not appear in PDL-based cultures, where cells with this morphology were extremely rare, although some round Nes⁺ cells were observed. On ECM, the presence of live mesenchymal cells greatly promoted GFAP expression, while the presence of MSC-CM was less effective (Figure 3A and Table 1), suggesting that the factors promoting astrocyte differentiation are likely short-lived. A similar result (weaker induction of GFAP by MSC-CM than by live MSC) was reported in another system [26] where MSC, plated at low density, were co-cultured with adult hippocampal neurosphere-derived neural stem cells in the presence of EGF and bFGF. In this system, cells were seeded on a poly-omithine/laminin or poly-lysine/laminin-substrate; however, these substrates were also briefly exposed to 10% serum to allow MSC attachment, which could add serum fibronectin to the coating. Most common protocols for culturing astrocytes include 10% FBS in the medium - which may mask the requirement for complex "ECM coating" for astrogenesis *in vitro.* The serum-free system described herein suggests this possibility.

Due to the lack of GFAP⁺ cell growth on PDL, it is not clear whether the soluble short-lived astrocyte-inducing factors required ECM for their signaling, or if the immature, round Nes⁺ cells simply did not express receptors for the inducing factors. Indeed, mesenchymal cell-derived factors TGFβ HGF, and BMPs were implicated in promoting astrogenesis [26, 43-45]; all these factors are ECM-bound in their inactive form and have short life span when released from ECM. Another illustration of the significance of mesenchymal cell-derived soluble and insoluble factors for astroglial differentiation comes from the observation of non-adherent cultures (Figure 7). Among all other tested differentiation markers, GFAP gene expression exhibited the most dramatic increase in neurospheres which were formed in the presence of MSC, compared to those formed in the presence of EFG and FGF2.

On ECM, astrocytes-like Nes⁺ cells that did not express GFAP were observed (Figure 3C). Their morphology implied that they may represent the radial glia, slowly dividing adult neural stem cells, which are GFAP-negative in rats [46-48]. This identity can be confirmed by phenotyping and, if confirmed, the assays described herein can be used to monitor the behavior of these adult stem cells in response to MSC.

Oligodendrocytic differentiation was monitored using an early oligodendrocytic marker, the myelin-processing enzyme CNPase, whose expression typically follows 04 expression and precedes the expression of myelin basic protein (MBP) [49]. In the experiments described herein, appearance of CNPase protein was detected relatively late after the appearance of its mRNA, but was expedited by the presence of MSC or SB623 cells (Figure 4). Quantifiable expression levels of CNPase mRNA were detected much earlier, and were directly MSC-dose-dependent (Figure 5). However, the dose-dependence curves became biphasic (Figure 6A) and eventually reversed. It appeared that while rat CNPase expression continued to increase with time in all cultures, at later time points lower doses of either MSC or SB623 cells, rather than higher ones, induced higher overall levels of CNPase mRNA. Protein staining confirmed this finding and revealed that low numbers of SB623 cells induced more intense CNPase staining than did 10 times more mesenchymal stem cells, while both doses increased numbers of dividing CNPase-positive cells (Figure 6B).

These results indicate the existence of a cell density-dependent inhibition of oligodendrocyte differentiation; however, it is unclear whether mesenchymal cells are responsible directly or indirectly. Expansion and differentiation of oligodendrocyte precursors are controlled by cell density [50, 51] and, although the control mechanism is unknown, it has been reported that local cell-to-cell interactions, rather than long range diffusible factors, were implicated; and that the effect is cell type-specific, *i.e.* it was mediated specifically by oligodendrocytic lineage [50]. The results of the assays described herein are consistent with the possibility that higher doses of mesenchymal cells inhibit oligodendrocyte differentiation indirectly, by increasing the proliferation of early oligodendrocyte precursors. On ECM, MSC-CM induced more than 3-fold higher CNPase expression than did live cells (Table 2), and much less GFAP expression was detected under these conditions. These observations suggest an interplay between, and balancing of, rates of proliferation and differentiation for astrocytes compared to oligodendrocytes. The data disclosed herein also supports the notion that ECM itself can play a role in promoting oligodendrocyte proliferation and differentiation [52]. Indeed, on PDL, even in the presence of MSC or MSC-CM, CNPase expression levels were low and the protein was not detected over the course of 2 weeks, while on ECM the protein was detected, even in the absence of other additives.

The methods and compositions disclosed herein enable the quantitative analysis of neuropoietic activity of test substances in mixed cross-species co-cultures. In this system, mesenchymal cell-derived ECM is used as a substrate for adherent co-culturing; neural cells are cultured in the same microenvironment from start to finish, without external growth factors; primary neural cells and test substances (e.g., MSC preparations) are co-cultured directly, at low cell plating density. The system allows analysis of secreted, diffusible, cell-associated and matrix-associated factors. Analysis can be conducted in a microplate format; using qRT-PCR-based readout for neural markers from total lysates.

Mesenchymal cell-derived ECM was chosen as a substrate for neural cell co-cultures based on previous observations that human MSC-derived ECM and, to a greater extent, SB623 cell-derived ECM, permit the growth of rat embryonic cortical cells and their subsequent differentiation to neuronal and glial lineages at relatively low cell plating densities and in the absence of growth factors [28]. Herein it is disclosed ECM coating created a favorable environment for Nestin-positive cell growth. The integral heparan sulfate proteoglycans (HSPG) of the ECM were important, since Heparinase 1 pre-treatment of ECM diminished nestin levels in cultures, in contrast to control-treated wells. The role of HSPGs suggested involvement of FGF2 signaling. Indeed, an antibody blocking FGF2, though not a control antibody, decreased nestin expression below basal levels in neural cultures. This suggests that FGF2 plays an important role in ECM-based cultures. FGF2 (of rat or human origin, or both) can provide physiological stimulation that supports the survival and the slow proliferation of neural stem/early precursor cells and enables the subsequent differentiation of the adherent culture. Recent reports identified mesenchymal cell-derived ECM as an integral part of an in vivo neural stem cell niche in the form of extravascular basal laminae (fractones) and its HSPGs were implicated in the accumulation of FGF2 [31, 32]. This observation justifies the use of mesenchymal ECM substrate for neural cell culturing to model a stem cell niche. Although most of the results disclosed herein were obtained using SB623-cell-derived ECM, MSC-ECM-based systems also produce similar results, although at longer culturing times.

When a cortical cell population is grown on ECM in the absence of growth factors or other test substances, differentiated glial cells are detected in 2-3 weeks [28]. In the presence of MSC, the neural population proliferated and differentiated significantly more rapidly, in an MSC-dose dependent manner (see Examples). The species-specific qRT-PCR readout method described herein is capable of detecting induction of rat neural markers in the presence of as little as 50 human MSC per 5000 rat neural cells. Levels of neural marker expression reflected a cumulative outcome of several processes in co-cultures. For example, an MSC-driven increase in total nestin expression (Figure 2) reflected increasing expression per cell, due to the growth of cellular cell extensions, increasing numbers of Nes⁺ stem cells (Nes⁺ colonies and dividing Nes⁺ cells), and increasing numbers of Nes⁺MAP2⁺ immature precursors (Example 1). MSC-driven increases in MAP2 or DCX expression noticeable in co-cultures at day 1 (Figure 2) were likely a result of MSC-enhanced neuritogenesis (Example 1 and [15, 22, 33, 34]). The second increase in neuronal markers was observed at later time points (at day 6 to 7), preceding the massive appearance of cells co-expressing both MAP2 and nestin proteins. These results are in agreement with previous reports, which demonstrated the stimulating effects of MSC on proliferation of neural precursors of neurosphere origin and on neuronal differentiation [23-25].

MSC are known to secrete many growth factors that have been shown to participate in the maintenance of neural precursors and neurodifferentiation *in vivo* [reviewed in 35] and the secretion of some of them, including BMP4, FGF2, EGF, VEGF, and PDGF-AA has been confirmed in some MSC batches used here (30 and co-owned US Patent Application Publication No. 2010/0266554). Blocking experiments demonstrated that MSC-produced FGF2 was the major factor responsible for MSC-driven nestin induction in co-cultures (Example 13). Nestin-inducing activity could also be efficiently transferred by MSC-CM; and approximately 85-90% of it could be removed from MSC-CM by immunoprecipitation of FGF2. The crucial role of FGF2 in the maintenance of neural stem cell is well known (reviewed in 36, 37); but the results described herein demonstrate that the contribution of MSC-derived FGF2 to MSC-driven nestin induction overwhelmed other possible contributors.

The induction of astrogenesis (measured as GFAP expression) under serum-free conditions is a hallmark of ECM-based cultures of E18 cortical cells. GFAP protein staining was closely associated with the staining for nestin filaments around day 7 (Example 2). Moreover, the formation of nestin filaments accompanying Nes⁺ cell spreading seemed to be a pre-requisite for astrocytic differentiation, since no GFAP induction was observed on other substrates that did not support Nes⁺-cell spreading (38). MSC greatly promoted GFAP expression. BMPs were found herein to be major mediators of this effect, since Noggin, a negative regulator of BMP activity, inhibited ∼90% of GFAP induction in co-cultures. BMP4 was abundantly expressed in MSC (39 and Table 4). An antibody that blocks human BMP4 eliminated ∼60% of GFAP induction, indicating that human BMP4 was a major astrogenic BMP. BMP4 was previously implicated in mediating astrogenic effects of specially induced rat MSC in co-cultures with mouse neurospheres (40). Example 14 shows that MSC-CM was less astrogenic than MSC, in agreement with a previous report (25). The residual astrogenic activity of MSC-CM could not be removed by immunodepleting BMP4; this could mean that BMP4 was not responsible for the residual astrogenic activity, or that it was present in an inactive form. However, MSC transfected with BMP4-siRNA, but not with control siRNA, when plated in co-cultures, showed reduced astrogenic activity, while FGF2 expression was not altered by the transfection (Example 14). Taken together, these results suggest that astrogenic activity of MSC is mediated in part by BMPs (specifically, human BMP4) and that the active BMP4 was either cell-associated or bound to the ECM, and not secreted into the medium. These results do not exclude the possibility that other MSC-derived factors, such as TGFβ, are involved (25, 42), although blocking TGFβ1 in co-cultures did not result in inhibition of astrogenesis.

Oligodendrocytic differentiation was monitored using an early oligodendrocytic marker, the myelin-processing enzyme CNPase, whose expression typically follows 04 expression, precedes the expression of myelin basic protein, and increases throughout the maturation process (43). In agreement with previous reports (24, 44, 45), oligodendrogenesis in the co-cultures described herein was clearly MSC-dependent; however, the timing of MSC-dose response was different from that of astrogenesis. On day 5 of co-culture, there was a direct relationship between MSC dose and levels of CNPse expression; whereas, on day 7, CNPase activation by increasing doses of MSC reached a plateau, beyond which further increases in MSC dose resulted in lower levels of activation. (Figure 5). Oligodendrocyte precursor expansion and differentiation are known to be controlled by cell density (46). Although the precise cell-density control mechanism is unknown, it has been reported that local cell-to-cell interactions between cells of oligodendrocytic lineage are responsible (47). The same biphasic dose-response of CNPase expression is observed at high doses of conditioned medium from MSC, indicating that the reversal of activation levels at high MSC doses is not due to the presence of high concentrations of mesenchymal cells. On the contrary, higher doses of MSC were very effective in inducing the proliferation of oligodendrocyte precursors; the precursors reached higher density more rapidly and suppressed their own differentiation (further accumulation of CNPase) earlier.

Disclosed herein is an *in vitro* system that enables the quantitative multifactorial analysis of the effects of a test substance on a primary neural cell population. The system preserves the complexity and some of the intrinsic interactions of a primary cell population. This system can be used to identify and quantitate soluble, cell-associated and/or matrix-bound neuropoietic factors and has been used to show the importance of soluble FGF2 and cell-associated or matrix-bound BMP4 in neuronal and astrocyte development, respectively. Finally, the system can be used for comparing the potencies of various lots of MSC or their derivatives (*e.g.,* SB623 cells), as well as for studying the effects of neural population on MSC.

The data presented herein suggest that SB623 cells induce the proliferation and differentiation of early neural precursors more efficiently than do their parental MSC.

In summary, the inventors have described an *in vitro* system that enables the imaging and the high-throughput quantitation of the effects of substances (such as, for example, MSC, SB623 cells and their products) on various stages of neural cell growth and differentiation. This system will facilitate the study of a number of differentiative processes, including, for example, MSC/neural cell interactions, and serve as a basis for potency assays for neuroregenerative cell-based therapies.

In addition, neurogenic effects of FGF2, and astrocytogenic effects of BMP4, have been demonstrated. Accordingly, FGF2 and BMP4 can be substituted for the neural precursor cells or for any of the neural cells described in co-owned U.S. Patent No. 7,682,825, for use in treatment of a disease, disorder or condition of the central or peripheral nervous system. Furthermore, FGF2 and BMP4 can be substituted for the neuronal precursor cells, the MASC-derived neuronal cells, or any of the graft-forming units described in co-owned U.S. Patent No. 8,092,792, for use in treatment of a central nervous system lesions (*e.g.,* ischemic stroke, hemorrhagic stroke).

### REFERENCES

1. Bianco P, Robey PG, Saggio I, Riminucci M (2010)."Mesenchymal" stem cells in human bone marrow (skeletal stem cells): a critical discussion of their nature, identity, and significance in incurable skeletal disease. Hum Gene Ther. 21:1057-66.
2. Torrente Y, Polli E. (2008). Mesenchymal stem cell transplantation for neurodegenerative diseases. Cell Transplant. 17:1103-13.
3. Qu C, Mahmood A, Lu D, Goussev A, Xiong Y, Chopp M. (2008). Treatment of traumatic brain injury in mice with marrow stromal cells. Brain Res. 1208:234-9.
4. Hofstetter CP, Schwarz EJ, Hess D, Widenfalk J, El Manira A, Prockop DJ, Olson L. (2002). Marrow stromal cells form guiding strands in the injured spinal cord and promote recovery. Proc Natl Acad Sci USA 99:2199-204.
5. Li Y, Chen J, Wang L, Zhang L, Lu M, Chopp M. (2001). Intracerebral transplantation of bone marrow stromal cells in a 1-methyl-4-phenyl-1,2,3,6-tetrahydropyridine mouse model of Parkinson's disease. Neurosci Lett. 316:67-70.
6. Li Y, Chen J, Zhang CL, Wang L, Lu D, Katakowski M, Gao Q, Shen LH, Zhang J, Lu M, Chopp M. (2005). Gliosis and brain remodeling after treatment of stroke in rats with marrow stromal cells. Glia. 49:407-17.
7. Chopp M, Zhang XH, Li Y, Wang L, Chen J, Lu D, Lu M, Rosenblum M. (2000). Spinal cord injury in rat: treatment with bone marrow stromal cell transplantation. Neuroreport 11:3001-3005
8. Glavaski-Joksimovic A, Virag T, Chang QA, West NC, Mangatu TA, McGrogan MP, Dugich-Djordjevic M, Bohn MC. (2009). Reversal of dopaminergic degeneration in a parkinsonian rat following micrografting of human bone marrow-derived neural progenitors. Cell Transplant. 18:801-14.
9. van Velthoven CT, Kavelaars A, van Bel F, Heijnen CJ. (2010). Repeated mesenchymal stem cell treatment after neonatal hypoxia-ischemia has distinct effects on formation and maturation of new neurons and oligodendrocytes leading to restoration of damage, corticospinal motor tract activity, and sensorimotor function. J Neurosci.30:9603-11.
10. Cova L, Armentero MT, Zennaro E, Calzarossa C, Bossolasco P, Busca G, Lambertenghi Deliliers G, Polli E, Nappi G, Silani V, Blandini F. (2010). Multiple neurogenic and neurorescue effects of human mesenchymal stem cell after transplantation in an experimental model of Parkinson's disease. Brain Res.1311:12-27
11. Yoo SW, Kim SS, Lee SY, Lee HS, Kim HS, Lee YD, Suh-Kim H. (2008). Mesenchymal stem cells promote proliferation of endogenous neural stem cells and survival of newborn cells in a rat stroke model. Exp Mol Med.40:387-97
12. Tfilin M, Sudai E, Merenlender A, Gispan I, Yadid G, Turgeman G. (2010). Mesenchymal stem cells increase hippocampal neurogenesis and counteract depressive-like behavior Mol Psychiatry. 15:1164-75
13. Munoz JR, Stoutenger BR, Robinson AP, Spees JL, Prockop DJ. (2005). Human stem/progenitor cells from bone marrow promote neurogenesis of endogenous neural stem cells in the hippocampus of mice. Proc Natl Acad Sci USA. 2005 102:18171-6.
14. Kan I, Barhum Y, Melamed E, Offen D. (2010). Mesenchymal Stem Cells Stimulate Endogenous Neurogenesis in the Subventricular Zone of Adult Mice. Stem Cell Rev. 2010 Sep 10. [Epub ahead of print]
15. Walker PA, Harting MT, Jimenez F, Shah SK, Pati S, Dash PK, Cox CS Jr (2010). Direct intrathecal implantation of mesenchymal stromal cells leads to enhanced neuroprotection via an NFkappaB-mediated increase in interleukin-6 production. Stem Cells Dev. 19:867-76.
16. Crigler L, Robey RC, Asawachaicham A, Gaupp D, Phinney DG. (2006). Human mesenchymal stem cell populations express a variety of neuro-regulatory molecules and promote neuronal cell survival and neuritogenesis Exp Neurology 198:54-64
17. Caplan AI, Dennis JE. (2006). Mesenchymal stem cells as trophic mediators. J Cell Biochem. 98:1076-84.
18. Chen X, Li Y, Wang L, Katakowski M, Zhang L, Chen J, Xu Y, Gautam SC, Chopp M. (2002). Ischemic rat brain extracts induce human marrow stromal cell growth factor production. Neuropathology. 22:275-9.
19. Nicaise C, Mitrecic D, Pochet R. (2010). Brain and spinal cord affected by amyotrophic lateral sclerosis induce differential growth factors expression in rat mesenchymal and neural stem cells. Neuropathol Appl Neurobiol. doi: 10.1111/j.1365-2990.2010.01124.x. [Epub ahead of print]
20. Mondal D, Pradhan L, LaRussa VF. (2004). Signal transduction pathways involved in the lineage-differentiation of NSCs: can the knowledge gained from blood be used in the brain? Cancer Invest. 22:925-43.
21. Garwood J, Rigato F, Heck N, Faissner A. (2001). Tenascin glycoproteins and the complementary ligand DSD-1-PG/ phosphacan--structuring the neural extracellular matrix during development and repair. Restor Neurol Neurosci. 19:51-64.
22. Kinnunen A, Niemi M, Kinnunen T, Kaksonen M, Nolo R, Rauvala H. (1999). Heparan sulphate and HB-GAM (heparin-binding growth-associated molecule) in the development of the thalamocortical pathway of rat brain. Eur J Neurosci. 11:491-502.
23. Lou S, Gu P, Chen F, He C, Wang M, Lu C. (2003). The effect of bone marrow stromal cells on neuronal differentiation of mesencephalic neural stem cells in Sprague-Dawley rats. Brain Res. 968:114-21.
24. Kang SK, Jun ES, Bae YC, Jung JS. (2003). Interactions between human adipose stromal cells and mouse neural stem cells in vitro. Brain Res Dev Brain Res. 145:141-9.
25. Bai L, Caplan A, Lennon D, Miller RH.(2007). Human mesenchymal stem cells signals regulate neural stem cell fate. Neurochem Res. 32:353-62.
26. Robinson AP, Foraker JE, Ylostalo J, Prockop DJ.(2010). Human Stem/Progenitor Cells from Bone Marrow Enhance Glial Differentiation of Rat Neural Stem Cells: A Role for Transforming Growth Factor β and Notch Signaling. Stem Cells Dev. 2010 Sep 14. [Epub ahead of print]
27. Campos LS. (2004). Neurospheres: insights into neural stem cell biology. J Neurosci Res. 78:761-9. Review.
28. Hack MA, Sugimori M, Lundberg C, Nakafuku M, Götz M. (2004). Regionalization and fate specification in neurospheres: the role of Olig2 and Pax6. Mol Cell Neurosci. 25:664-78.
29. Aizman I, Tate CC, McGrogan M, Case CC. (2009). Extracellular matrix produced by bone marrow stromal cells and by their derivative, SB623 cells, supports neural cell growth. J Neurosci Res. 87:3198-206.
30. Lathia JD, Rao MS, Mattson MP, Ffrench-Constant C. (2007). The microenvironment of the embryonic neural stem cell: lessons from adult niches? Dev Dyn. 236:3267-82.
31. Pierret C, Morrison JA, Rath P, Zigler RE, Engel LA, Fairchild CL, Shi H, Maruniak JA, Kirk MD. (2010). Developmental cues and persistent neurogenic potential within an in vitro neural niche. BMC Dev Biol. 10:5-23
32. Goetz AK, Scheffler B, Chen HX, Wang S, Suslov O, Xiang H, Brüstle O, Roper SN, Steindler DA. (2006). Temporally restricted substrate interactions direct fate and specification of neural precursors derived from embryonic stem cells. Proc Natl Acad Sci USA. 103:11063-8.
33. Mercier F, Kitasako JT, Hatton GI (2002). Anatomy of the brain neurogenic zones revisited: fractones and the fibroblast/macrophage network. J Comp Neurol. 451:170-88.
34. Kerever A, Schnack J, Vellinga D, Ichikawa N, Moon C, Arikawa-Hirasawa E, Efird JT, Mercier F. (2007). Novel extracellular matrix structures in the neural stem cell niche capture the neurogenic factor fibroblast growth factor 2 from the extracellular milieu Stem Cells. 25:2146-57.
35. Wagner W, Wein F, Seckinger A, Frankhauser M, Wirkner U, Krause U, Blake J, Schwager C, Eckstein V, Ansorge W, Ho AD. (2005). Comparative characteristics of mesenchymal stem cells from human bone marrow, adipose tissue, and umbilical cord blood. Exp Hematol. 33:1402-16.
36. Chen XD, Dusevich V, Feng JQ, Manolagas SC, Jilka RL. (2007). Extracellular matrix made by bone marrow cells facilitates expansion of marrow-derived mesenchymal progenitor cells and prevents their differentiation into osteoblasts. J Bone Miner Res. 22:1943-56.
37. Nagase T, Ueno M, Matsumura M, Muguruma K, Ohgushi M, Kondo N, Kanematsu D, Kanemura Y, Sasai Y. (2009). Pericellular matrix of decidua-derived mesenchymal cells: a potent human-derived substrate for the maintenance culture of human ES cells. Dev Dyn. 238:1118-30.
38. Wang Y, Tu W, Lou Y, Xie A, Lai X, Guo F, Deng Z. (2009). Mesenchymal stem cells regulate the proliferation and differentiation of neural stem cells through Notch signaling. Cell Biol Int. 33:1173-9.
39. Tate CC, Fonck C, McGrogan M, Case CC. (2010). Human mesenchymal stromal cells and their derivative, SB623 cells, rescue neural cells via trophic support following in vitro ischemia. Cell Transplant. 19:973-84.
40. Führmann T, Montzka K, Hillen LM, Hodde D, Dreier A, Bozkurt A, Wöltje M, Brook GA. (2010). Axon growth-promoting properties of human bone marrow mesenchymal stromal cells. Neurosci Lett. 474:37-41.
41. Kamei N, Tanaka N, Oishi Y, Ishikawa M, Hamasaki T, Nishida K, Nakanishi K, Sakai N, Ochi M. (2007). Bone marrow stromal cells promoting corticospinal axon growth through the release of humoral factors in organotypic cocultures in neonatal rats. J Neurosurg Spine 6:412-9.
42. Goetschy JF, Ulrich G, Aunis D, Ciesielski-Treska J. (1987). Fibronectin and collagens modulate the proliferation and morphology of astroglial cells in culture. Int J Dev Neurosci. 5:63-70
43. Stipursky J, Gomes FC. (2007). TGF-betal/SMAD signaling induces astrocyte fate commitment in vitro: implications for radial glia development. Glia. 55:1023-33.
44. Wislet-Gendebien S, Bruyère F, Hans G, Leprince P, Moonen G, Rogister B. (2004). Nestin-positive mesenchymal stem cells favour the astroglial lineage in neural progenitors and stem cells by releasing active BMP4. BMC Neurosci. 5:33-44
45. Imura T, Tane K, Toyoda N, Fushiki S. (2008). Endothelial cell-derived bone morphogenetic proteins regulate glial differentiation of cortical progenitors. Eur J Neurosci. 27:1596-606.
46. Chojnacki AK, Mak GK, Weiss S. (2009). Identity crisis for adult periventricular neural stem cells: subventricular zone astrocytes, ependymal cells or both? Nat Rev Neurosci.10:153-63.
47. Alvarez-Buylla A, Garcia-Verdugo JM, Tramontin AD. (2001). A unified hypothesis on the lineage of neural stem cells. Nat Rev Neurosci. 2:287-93.
48. Sancho-Tello M, Vallés S, Montoliu C, Renau-Piqueras J, Guerri C. (1995). Developmental pattern of GFAP and vimentin gene expression in rat brain and in radial glial cultures. Glia 15:157-66.
49. Pfeiffer SE, Warrington AE, Bansal R. (1993). The oligodendrocyte and its many cellular processes. Trends Cell Biol. 3:191-7.
50. Zhang H, Miller RH. (1996). Density-dependent feedback inhibition of oligodendrocyte precursor expansion. J Neurosci. 16:6886-95.
51. Hugnot JP, Mellodew K, Pilcher H, Uwanogho D, Price J, Sinden JD. (2001). Direct cell-cell interactions control apoptosis and oligodendrocyte marker expression of neuroepithelial cells. J Neurosci Res.65:195-207.
52. Hu J, Deng L, Wang X, Xu XM. (2009). Effects of extracellular matrix molecules on the growth properties of oligodendrocyte progenitor cells in vitro. J Neurosci Res. 87:2854-62.

## Claims

1. Use of a culture system for quantitative functional assays for measuring the effects of substances on neural cells comprising:
(a) a solid substrate with an extracellular matrix deposited thereon, wherein the extracellular matrix is deposited by:
(i) a mesenchymal stem cell (MSC), or
(ii) a MSC that has been transfected with a nucleic acid, wherein the nucleic acid encodes a Notch intracellular domain but does not encode full-length Notch protein;
(b) embryonic cortical cells; and
(c) a test substance,
wherein the test substance is a chemical compound, a polypeptide, a test cell, a test cell culture or test conditioned medium from a cell culture.

2. The use of the culture system of claim 1, wherein the MSC is a MSC obtained from a human.

3. The use of the culture system of claim 1, wherein the solid substrate is a substrate selected from the group consisting of plastic, nitrocellulose and glass.

4. The use of the culture system of claim 1, wherein the embryonic cortical cells are embryonic cortical cells obtained from a mouse or a rat.

5. The use of the culture system of claim 1, wherein the test cell is a cell selected from the group consisting of:
(a) a mesenchymal stem cell, and
(b) a descendant of a mesenchymal stem cell that has been transfected with a nucleic acid encoding a Notch intracellular domain.

6. Use of a kit for determining the effect of a substance on neuropoiesis, neurogenesis, astrocytogenesis, or oligodendrocytogenesis; the kit comprising:
(a) the culture system of claim 1; and
(b) one or more reagents for detection of a neuronal or glial marker molecule.

7. The use of the kit of claim 6, wherein the detection is by immunohistochemistry or quantitative reverse transcriptase polymerase chain reaction (qRT-PCR) and the reagent comprises one or more antibodies specific to the neuronal or glial marker molecule, or one or more oligonucleotide primers or oligonucleotide probes.

8. The use of the kit of claim 7, wherein the neuronal or glial marker molecule is a molecule selected from the group consisting of one or more of microtubule-associated protein 2 (MAP2), doublecortin
(DCX), beta-tubulin type III (TuJ1), synaptophysin, neuron-specific enolase, glial fibrillary acidic protein (GFAP), Glast, glutamine synthetase, 2', 3'-cyclic nucleotide 3' phosphodiesterase
(CNPase), the O1 antigen, the 04 antigen, myelin basic protein, oligodendrocyte transcription factor 1, oligodendrocyte transcription factor 2, oligodendrocyte transcription factor 3, NG2, and
myelin-associated glycoprotein.

9. A method for testing for a substance that promotes neurogenesis, gliogenesis,
growth of neural precursor cells (NPCs) or differentiation of NPCs, the method comprising:
measuring the effect of the substance in the culture system of any of claims 1 to 5 on one or more of:
(a) growth of neurons;
(b) growth of glial cells;
(c) growth of NPCs; or
(d) differentiation of NPCs;
wherein growth of neurons indicates that the substance promotes neurogenesis, growth of glial cells indicates the substance promotes gliogenesis, growth of NPCs indicates that the substance promotes the growth of NPCs and differentiation of NPCS indicates that
the substance promotes the differentiation of NPCs.

10. The method of claim 9, wherein the substance is a chemical compound, a polypeptide, a test cell, a test cell culture or test conditioned medium from a cell culture.

11. The method of claim 10, wherein the test cell is selected from the group consisting of
(a) a mesenchymal stem cell, and
(b) a descendant of a mesenchymal stem cell that has been transfected with a nucleic acid encoding a Notch intracellular domain.

12. The method of claim 10, wherein the neurogenesis, gliogenesis, growth of NPCs or differentiation of NPCs is promoted by a protein expressed on the surface of the test cell.

13. The method of claim 9, wherein:
(a) growth of neurons is measured by neurite outgrowth or by expression of a marker selected from the group consisting of microtubule-associated protein 2 (MAP2), doublecortin (DCX), beta-tubulin type III (TuJ1), synaptophysin and neuron-specific enolase;
(b) the glial cells are astrocytes and growth of the astrocytes is measured by expression of glial fibrillary acidic protein (GFAP), Glast, or glutamine synthetase;
(c) the glial cells are oligodendrocytes and growth of the oligodendrocytes is measured by expression a marker selected from the group consisting of 2', 3'-cyclic nucleotide 3'
phosphodiesterase (CNPase), the O1 antigen, the O4 antigen, myelin basic protein, oligodendrocyte transcription factor 1, oligodendrocyte transcription factor 2, oligodendrocyte transcription factor 3, NG2, and myelin-associated glycoprotein;
(d) growth of NPCs is measured by expression of nestin, Glast or SOX2; or
(e) differentiation of NPCs is evidenced by neurite outgrowth, or by expression of a marker selected from the group consisting of microtubule-associated protein 2 (MAP2), doublecortin (DCX), beta-tubulin type III (TuJ1), synaptophysin, neuron-specific enolase, glial
fibrillary acidic protein (GFAP), glutamine synthetase, the GLAST glutamate transporter, 2', 3'-cyclic nucleotide 3'phosphodiesterase (CNPase), the O1 antigen, the O4 antigen, myelin basic
protein, oligodendrocyte transcription factor 1, oligodendrocyte transcription factor 2, oligodendrocyte transcription factor 3, NG2, and myelin-associated glycoprotein.

14. The method of claim 9, wherein the growth of neurons or glial cells is compared to growth or neurons or glial cells, respectively, in the absence of the substance; or wherein the growth or differentiation of NPCs is compared to the growth or differentiation,
respectively,
of NPCs in the absence of the substance.

## Patentansprüche

1. Verwendung eines Kultursystems für quantitative funktionelle Assays zum Messen der Wirkungen von Substanzen auf neurale Zellen, umfassend:
(a) ein Feststoffsubstrat mit einer darauf abgeschiedenen extrazellulären Matrix, wobei die extrazelluläre Matrix abgeschieden wird durch:
(i) eine mesenchymale Stammzelle (MSC), oder
(ii) eine mesenchymale Stammzelle, die mit einer Nukleinsäure transfiziert ist, wobei die Nukleinsäure eine intrazelluläre Notch-Domäne kodiert, aber nicht ein Notch-Protein mit voller Länge kodiert;
(b) embryonale kortikale Zellen; und
(c) eine Testsubstanz,
wobei die Testsubstanz eine chemische Verbindung, ein Polypeptid, eine Testzelle, eine Test-Zellkultur oder ein durch den Test konditioniertes Medium einer Zellkultur ist.

2. Verwendung des Kultursystems nach Anspruch 1, wobei die MSC eine von einem Menschen gewonnene MSC ist.

3. Verwendung des Kultursystems nach Anspruch 1, wobei das feste Substrat ein Substrat ist, ausgewählt aus der Gruppe bestehend aus Kunststoff, Nitrocellulose und Glas.

4. Verwendung des Kultursystems nach Anspruch 1, wobei die embryonalen kortikalen Zellen embryonale kortikale Zellen sind, die von einer Maus oder einer Ratte gewonnen sind.

5. Verwendung des Kultursystems nach Anspruch 1, wobei die Testzelle eine Zelle ist, ausgewählt aus der Gruppe bestehend aus:
(a) einer mesenchymalen Stammzelle, und
(b) eines Abkömmlings einer mesenchymalen Stammzelle, die mit einer Nukleinsäure transfiziert wurde, die eine intrazelluläre Notch-Domäne kodiert.

6. Verwendung eines Kits zum Bestimmen der Wirkung einer Substanz auf Neuropoisese, Neurogenese, Astrozytogenese oder Oligodendrozytogenese; wobei das Kit Folgendes umfasst:
(a) das Kultursystem nach Anspruch 1; und
(b) ein oder mehrere Reagenzien zum Nachweis eines neuronalen oder glialen Markermoleküls.

7. Verwendung des Kits nach Anspruch 6, wobei der Nachweis durch Immunhistochemie oder quantitative Reverse-Transkriptase-Polymerase-Kettenreaktion (qRT-PCR) erfolgt und das Reagenz einen oder mehrere Antikörper umfasst, die spezifisch sind für ein neuronales oder glialesMarkermolekül, oder eine oder mehrere Oligonukleotid-Primer oder Oligonukleotid-Sonden.

8. Verwendung des Kits nach Anspruch 7, wobei das neuronale oder glialeMarkermalekül ein Molekül darstellt, ausgewählt aus der Gruppe bestehend aus einem oder mehreren Mikrotubuli-assoziiertem Protein 2 (MAP2), Doublecortin (DCX), Beta-Tubulin Typ III (TuJ1), Synaptophysin, neuronenspezifischer Enolase, saurem Gliafaserprotein (GFAP), Glast, Glutaminsynthetase, 2',3-Cyclonukleotid-3'-Phosphodiesterase (CNPase), dem O1-Antigen dem O4.-Antigen, basischem Myelinprotein, Oligodendrozytentranskriptionsfaktor 1, Oligodendrozytentranskriptionsfaktor 2, Oligodendrozytentranskriptionsfaktor 3, NG2 und Myelin-assoziiertem Glykoprotein.

9. Verfahren zum Testen auf eine Substanz, die die Neurogenese, Gliogenese, das Wachstum neuronaler Precursorzellen (NPCs) oder die Differenzierung von NPCs fördert, wobei das Verfahren umfasst:
Messen der Wirkung der Substanz im Kultursystem nach einem der Ansprüche 1 bis 5 auf ein oder mehrere von:
(a) das Wachstum von Neuronen;
(b) das Wachstum von Glialzellen;
(c) das Wachstum von NPCs; oder
(d) die Differenzierung von NPCs;
wobei das Wachstum der Neuronen darauf hinweist, dass die Substanz die Neurogenese, fördert, das Wachstum von Glialzellen darauf hinweist, dass die Substanz die Gliogenese fördert, das Wachstum der NPCs darauf hinweist, dass die Substanz das Wachstum der NPCs fördert, und die Differenzierung der NPCs darauf hinweist, dass die Substanz die Differenzierung der NPCs fördert.

10. Verfahren nach Anspruch 9, wobei die Substanz eine chemische Verbindung, ein Polypeptid, eine Testzelle, eine Testzellkultur oder ein durch den Test konditioniertes Medium einer Zellkultur ist.

11. Verfahren nach Anspruch 10, wobei die Testzelle ausgewählt wird aus der Gruppe bestehend aus
(a) einer mesenchymalen Stammzelle, und
(b) eines Abkömmlings einer mesenchymalen Stammzelle, die mit einer Nukleinsäure transfiziert ist, die eine intrazelluläre Notch-Domäne kodiert.

12. Verfahren nach Anspruch 10, wobei die Neurogenese, Gliogenese, das Wachstum der NPCs oder die Differenzierung der NPCs durch ein Protein gefördert wird, das auf der Oberfläche der Testzelle exprimiert wird.

13. Verfahren nach Anspruch 9, wobei:
(a) das Wachstum der Neuronen anhand des Neurit-Auswuchs oder durch die Expression eines Markers gemessen wird, ausgewählt aus der Gruppe bestehend aus einem Mikrotubuli-assoziiertem Protein 2 (MAP2), Doublecortin (DCX), Beta-Tubulin Typ III (TuJ1), Synaptophysin und neuronenspezifischer Enolase;
(b) die Glialzellen Astrozyten sind und das Wachstum der Astrozyten durch Expression des sauren Gliafaserproteins (GFAP), Glast oder Glutaminsynthetase gemessen wird;
(c) die Glialzellen Oligodendrozyten sind und das Wachstum der Oligodendrozyten
anhand der Expression des Markers gemessen wird, der ausgewählt wird aus der Gruppe bestehend aus 2',3'-Cyclonukleotid-3'-Phosphodiesterase (CNPase), dem O1-Antigen dem O4.-Antigen, dem basischen Myelinprotein, dem Oligodendrozytentranskriptionsfaktor 1, Oligodendrozytentranskriptionsfaktor 2, Oligodendrozytentranskriptionsfaktor 3, NG2 und dem myelin-assoziierten Glykoprotein;
(d) das Wachstum der NPCs anhand der Expression von Nestin, Glast oder SOX2 gemessen wird; oder
(e) die Differenzierung der NPCs durch den Neurit-Auswuchs nachgewiesen wird, oder durch Expression eines Markers, ausgewählt aus der Gruppe bestehend aus Mikrotubuli-assoziiertem Protein 2 (MAP2), Doublecortin (DCX), Beta-Tubulin Typ III, Synatophysin, neuronenspezifische Enolase, saurem Gliafaserprotein (GFAP), Glutaminsynthetase, dem GLAST-Glutamattransporter, 2',3-Cyclonukleotid-3'-Phosphodiesterase (CNPase), dem O1-Antigen dem O4.-Antigen, dem basischen Myelinprotein, dem Oligodendrozytentranskriptionsfaktor 1, Oligodendrozytentranskriptionsfaktor 2, Oligodendrozytentranskriptionsfaktor 3, NG2 und dem Myelin-assoziiertem Glykoprotein.

14. Verfahren nach Anspruch 9, wobei das Wachstum der Neuronen oder Gliazellen jeweils mit dem Wachstum der Neuronen oder Gliazellen in Abwesenheit der Substanz verglichen wird; oder wobei das Wachstum oder die Differenzierung der NPCs jeweils mit dem Wachstum oder der Differenzierung der NPCs in Abwesenheit der Substanz verglichen wird.

## Revendications

1. Utilisation d'un système de culture pour des dosages fonctionnels quantitatifs destinés à mesurer les effets de substances sur des cellules neuronales, comprenant :
(a) un substrat solide sur lequel est reçue une matrice extracellulaire, dans laquelle la matrice extracellulaire est reçue par :
(i) une cellule souche mésenchymateuse (MSC), ou
(ii) une MSC ayant été transfectée avec un acide nucléique, dans laquelle l'acide nucléique code pour un domaine intracellulaire de Notch mais ne code pas pour une protéine Notch de longueur complète ;
(b) des cellules corticales embryonnaires, et
(c) une substance de test,
dans laquelle la substance de test est un composé chimique, un polypeptide, une cellule de test, une culture cellulaire de test, ou un milieu conditionné pour des tests provenant d'une culture cellulaire.

2. Utilisation du système de culture selon la revendication 1, dans laquelle la MSC est une MSC obtenue à partir d'un être humain.

3. Utilisation du système de culture selon la revendication 1, dans laquelle le substrat solide est un substrat sélectionné parmi le groupe consistant en du plastique, du nitrate de cellulose, et du verre.

4. Utilisation du système de culture selon la revendication 1, dans laquelle les cellules corticales embryonnaires sont des cellules corticales embryonnaires obtenues à partir d'une souris ou d'un rat.

5. Utilisation du système de culture selon la revendication 1, dans laquelle la cellule de test est une cellule sélectionnée parmi le groupe consistant en :
(a) une cellule souche mésenchymateuse, et
(b) une descendance d'une MSC ayant été transfectée avec un acide nucléique codant pour un domaine intracellulaire de Notch.

6. Utilisation d'un kit pour déterminer l'effet d'une substance sur une neuropoièse, une neurogénèse, une astrocytogénèse, ou une oligodendrocytogénèse, le kit comprenant :
(a) le système de culture selon la revendication 1, et
(b) un ou plusieurs réactifs pour la détection d'une molécule de marqueur neuronal ou glial.

7. Utilisation du kit selon la revendication 6, dans laquelle la détection s'effectue par immunohistochimie ou par une amplification en chaîne par polymérase à transcriptase inverse quantitative (qRT-PCR), et le réactif comprend un ou plusieurs anticorps spécifiques à la molécule de marqueur neuronal ou glial, ou une ou plusieurs amorces d'oligonucléotide ou sondes d'oligonucléotide.

8. Utilisation du kit selon la revendication 7, dans laquelle la molécule de marqueur neuronal ou glial est une molécule sélectionnée parmi le groupe consistant en un ou plusieurs éléments parmi une protéine associée aux microtubules 2 (MAP2), du Doublecortin (DCX), de la bêta-tubuline de type III (TuJ1), de la synaptophysine, une énolase spécifique aux neurones, une protéine acide fibrillaire gliale (GFAP), du Glast, la glutamine synthétase, de la 2',3'-nuc!éotide cyclique 3' phosphodiestérase (CNPase), l'antigène O1, l'antigène O4, une protéine de base de myéline, le facteur de transcription 1 d'oligodendrocyte, le facteur de transcription 2 d'oligodendrocyte, le facteur de transcription 3 d'oligodendrocyte, NG2, et une glycoprotéine associée à la myéline.

9. Procédé destiné à tester quant à une substance de promotion de la neurogénèse, de la gliogénèse, de la croissance de cellules précurseurs neuronales (NPC), ou de la différentiation de NPC, le procédé comprenant les étapes consistant à :
mesurer l'effet de la substance dans le système de culture selon l'une quelconque des revendications 1 à 5 sur un ou plusieurs des éléments suivants :
(a) la croissance de neurones ;
(b) la croissance de cellules gliales ;
(c) la croissance de NPC, ou
(d) la différentiation de NPC,
dans lequel la croissance de neurones indique que la substance promeut la neurogénèse ; la croissance de cellules de cellules gliales indique que la substance promeut la gliogénèse ; la croissance de NPC indique que la substance promeut la croissance de NPC, et la différentiation de NPC indique que la substance promeut la différentiation de NPC.

10. Procédé selon la revendication 9, dans lequel la substance de test est un composé chimique, un polypeptide, une cellule de test, une culture cellulaire de test, ou un milieu conditionné pour des tests provenant d'une culture cellulaire.

11. Procédé selon la revendication 10, dans lequel la cellule de test est sélectionnée parmi le groupe consistant en :
(a) une cellule souche mésenchymateuse, et
(b) une descendance d'une cellule souche mésenchymateuse ayant été transfectée avec un acide nucléique codant pour un domaine intracellulaire de Notch.

12. Procédé selon la revendication 10, dans lequel la neurogénèse, la gliogénèse, la croissance de NPC, ou la différentiation de NPC sont promues par une protéine exprimée sur la surface de la cellule de test.

13. Procédé selon la revendication 9, dans lequel :
(a) la croissance de neurones est mesurée par une excroissance de neurite ou par l'expression d'un marqueur sélectionné parmi le groupe consistant en une protéine associée aux microtubules 2 (MAP2), du Doublecortin (DCX), une bêta-tubuline de type III (TuJ1), de la synaptophysine, une énolase spécifique aux neurones ;
(b) les cellules gliales sont des astrocytes et la croissance des astrocytes est mesurée par l'expression d'une protéine acide fibrillaire gliale (GFAP), de Glast, ou de glutamine synthétase ;
(c) les cellules gliales sont des oligodendrocytes et la croissance des oligodendrocytes est mesurée par l'expression d'un marqueur sélectionné parmi le groupe consistant en une 2',3'-nucléotide cyclique 3' phosphodiestérase (CNPase), l'antigène O1, l'antigène O4, une protéine de base de myéline, le facteur de transcription 1 d'oligodendrocyte, le facteur de transcription 2 d'oligodendrocyte, le facteur de transcription 3 d'oligodendrocyte, NG2, et une glycoprotéine associée à la myéline ;
(d) la croissance de NPC est mesurée par l'expression de nestine, de Glast, ou de SOX2, ou
(e) la différentiation de NPC est attestée par une excroissance de neurite, ou par l'expression d'un marqueur sélectionné parmi le groupe consistant en une protéine associée aux microtubules 2 (MAP2), du Doublecortin (DCX), de la bêta-tubuline de type III (TuJ1), de la synaptophysine, une énolase spécifique aux neurones, une protéine acide fibrillaire gliale (GFAP), de la glutamine synthétase, le transporteur de glutamate GLAST, une 2',3'-nucléotide cyclique 3' phosphodiestérase (CNPase), l'antigène O1, l'antigène O4, la protéine de base de myéline, le facteur de transcription 1 d'oligodendrocyte, le facteur de transcription 2 d'oligodendrocyte, le facteur de transcription 3 d'oligodendrocyte, NG2, et une glycoprotéine associée à la myéline.

14. Procédé selon la revendication 9, dans lequel la croissance de neurones ou de cellules gliales est comparée respectivement à la croissance de neurones ou de cellules gliales en l'absence de la substance, ou dans lequel la croissance ou différentiation des NPC est respectivement comparée à la croissance ou différentiation de NPC en l'absence de la substance.
